# EUROPÄISCHE PATENTSCHRIFT

(11) **EP 3 293 215 B1**
(45) Veröffentlichungstag und Bekanntmachung des Hinweises auf die Patenterteilung: **21.10.2020**
(21) Anmeldenummer: 16187716.2
(22) Anmeldetag: 07.09.2016
(51) Int. Cl.: A61K 6/00, A61K 6/62, A61K 6/887, C08F 20/10, C08F 2/50

(54) **POLYMERISIERBARE ZUSAMMENSETZUNGEN MIT ACYLZINN-PHOTOINITIATOREN**
POLYMERIZABLE COMPOSITIONS HAVING ACYL TIN PHOTOINITIATORS
COMPOSITIONS POLYMÉRISABLES AVEC PHOTOINITIATEURS ACYLZINN

(43) Veröffentlichungstag der Anmeldung: 14.03.2018
(73) Patentinhaber: Ivoclar Vivadent AG, 9494 Schaan (LI); Technische Universität Wien, 1040 Wien (AT)
(72) Erfinder: Mozner, Norbert, 9495 Triesen (LI); Fischer, Urs Karl, 9320 Arbon (CH); Burtscher, Peter, 6830 Rankweil (AT); Liska, Robert, 2123 Schleinbach (AT); Knaack, Patrick, 1160 Wien (AT); Mitterbauer, Moritz, 1090 Wien (AT)
(74) Vertreter: Uexküll & Stolberg

(56) Entgegenhaltungen:
- EP-A1- 2 103 297
- EP-A2- 0 405 786
- US-A1- 2005 282 700
- MOSZNER N ET AL: "Benzoyl germanium derivatives as novel visible light photoinitiators for dental materials", DENTAL MATERIALS, ELSEVIER, AMSTERDAM, NL, Bd. 24, Nr. 7, 1. Juli 2008 (2008-07-01), Seiten 901-907, XP022671145, ISSN: 0109-5641, DOI: 10.1016/J.DENTAL.2007.11.004 [gefunden am 2007-12-21]

## Beschreibung

Die vorliegende Erfindung betrifft polymerisierbare Zusammensetzungen, die eine Acylzinn-Verbindung als Polymerisationsinitiator enthalten. Die Zusammensetzungen eignen sich besonders zur Herstellung von Adhäsiven, Beschichtungen, Zementen, Kompositen, Formteilen wie Stäben, Platten, Scheiben oder Linsen und insbesondere von Dentalwerkstoffen und Werkstoffen für die stereolithographische Herstellung von Formteilen.

Für die Aushärtung von photopolyreaktionsfähigen Harzen spielt der eingesetzte Photoinitiator eine entscheidende Rolle. Bei Bestrahlung mit UV- oder sichtbarem Licht absorbiert dieser Licht und bildet die polymerisationsauslösenden Spezies. Im Fall der radikalischen Polymerisation handelt es sich dabei um freie Radikale. Basierend auf dem chemischen Mechanismus der Radikalbildung werden die Photoinitiatoren in zwei Klassen eingeteilt.

Norrish-Typ-I-Photoinitiatoren bilden beim Bestrahlen durch eine unimolekulare Bindungsspaltung freie Radikale. Norrish-Typ-II-Photoinitiatoren durchlaufen bei der Bestrahlung eine bimolekulare Reaktion, wobei der Photoinitiator im angeregten Zustand mit einem zweiten Molekül, dem Coinitiator reagiert und sich durch Elektronen- und Protonentransfer die polymerisationsauslösenden Radikale bilden. Für die UV-Lichthärtung werden Typ-I- und Typ-II-Photoinitiatoren eingesetzt, für den sichtbaren Lichtbereich kommen bisher, abgesehen von Bisacyldialkylgermanium-Verbindungen, nahezu ausschliesslich Typ-II-Photoinitiatoren zum Einsatz.

Die UV-Härtung zeichnet sich durch eine hohe Reaktionsgeschwindigkeit aus und wird häufig für die Beschichtungen von unterschiedlichen Substraten wie z.B. Holz, Metall oder Glas eingesetzt. So wird zum Beispiel in EP 1 247 843 A2 ein UV-härtendes Beschichtungsmaterial beschrieben, bei dem Typ-I-Photoinitiatoren wie Diethoxyphenylacetophenon oder Acyl- oder Bisacylphosphinoxide zum Einsatz kommen.

Die WO 01/51533 A1 beschreibt ein UV-härtendes Holzbeschichtungsmaterial, bei dem ebenfalls Acylphosphinoxide, α-Hydroxyalkylphenone oder α-Dialkoxyacetophenone als Photoinitiatoren Anwendung finden. Bedingt durch die geringe Wellenlänge des UV-Lichts lassen sich mit der UV-Härtung vor allem transparente Beschichtungen mit geringer Schichtstärke realisieren. Bei starker Einfärbung oder Pigmentierung, in gefüllten Systemen und bei grösseren Schichtstärken kommt man an die Grenzen der UV-Härtung. Derartige photopolyreaktionsfähigen Harze mit deutlich reduzierter Transparenz härten mit UV-Licht nur unvollständig aus.

Werden grössere Durchhärtungstiefen benötigt, wie zum Beispiel bei der Aushärtung von lichthärtenden Dentalfüllungsmaterialien, so wird mit sichtbarem Licht bestrahlt. Das dafür am häufigsten eingesetzte Photoinitiatorsystem ist eine Kombination aus einem α-Diketon mit einem Amincoinitiator, wie sie z.B. in GB 1 408 265 beschrieben ist.

Dentalzusammensetzungen, in denen dieses Photoinitiatorsystem eingesetzt wird, werden z.B. in der US 4,457,818 oder der US 4,525,256 offenbart, wobei vorzugsweise Campherchinon als α-Diketon eingesetzt wird. Campherchinon hat ein Absorptionsmaximum bei einer Wellenlänge von 468 nm. Dadurch zeigt Campherchinon eine starke Gelbfärbung, mit dem Nachteil, dass mit Campherchinon/Amin initiierte Materialien nach der Aushärtung häufig einen Gelbstich aufweisen, da das Initiatorsystem nicht vollständig ausbleicht (N. Moszner, R. Liska, Photoinitiators for direct adhesive restorative materials, In: Basics and Applications of Photopolymerization Reactions, Vol. 1; Fouassier, J.-P., Allonas, X., Eds., Research Signpost, Kerala, 2010, 93-114). Dieses Bleichungsverhalten ist vor allem bei hellen Weisstönen des auspolymerisierten Materials sehr nachteilig. Ausserdem besitzen Campherchinon-Amin-Systeme bei Anwendung in sauren Adhäsiven den Nachteil, dass die radikalbildende Aminkomponente protoniert und dadurch für die Radikalbildung teilweise deaktiviert wird.

Die Verwendung von Germaniumverbindungen als Photoinitiatoren ist bekannt. Vor allem Bisacyldialkylgermaniumverbindungen sind effiziente Norrish-Typ-I Photoinitiatoren für die Aushärtung im Blaulichtbereich (B. Ganster, U. K. Fischer, N. Moszner, R. Liska, New photocleavable structures, Diacylgerman-based photoinitiators for visible light curing, Macromolecules 41 (2008) 2394-2400; N. Moszner, U.K. Fischer, B. Ganster, R. Liska, V. Rheinberger, Benzoyl germanium derivatives as novel visible light photoinitiators for dental materials Dent. Mater. 24 (2008) 901-907; N. Moszner, F. Zeuner, I. Lamparth, U. K. Fischer, Benzoylgermanium derivatives as novel visible-light photoinitiators for dental composites, Macromol. Mater. Eng. 294 (2009) 877-886).

EP 1 905 413 A1 und EP 1 905 415 A1 offenbaren Mono-, Bis- und Triacylgermaniumverbindungen, die sich als Photoinitiatoren zur Härtung von Dentalwerkstoffen mit sichtbarem Licht eignen. Ihre Synthese ist aufwendig und erfolgt ausgehend von teuren Dialkylgermaniumdihalogeniden unter Nutzung der Dithianschutzgruppentechnik und säulenchromatographischer Reinigung.

Aus der EP 2 103 297 A1 sind als Photoinitiatoren geeignete Acylgermanium-Verbindungen bekannt, die mehrere Germaniumatome enthalten.

Die WO 2015/067815 A1 offenbart Bis(germyl)ketone der Formel R¹R²R³Ge(CO)GeR⁴R⁵R⁶ und Verfahren zu deren Herstellung. Diese Bis(germyl)ketone sollen sich ebenfalls als Photoinitiatoren für Dentalwerkstoffe eignen.

Der Erfindung liegt die Aufgabe zugrunde, polymerisierbare Zusammensetzungen bereitzustellen, die sich mit Licht im sichtbaren Bereich härten lassen, die sich durch eine hohe Reaktivität und gute Härtungscharakteristik sowie ein gutes Bleachingverhalten auszeichnen und die sich insbesondere durch sichtbares Licht im langwelligen Bereich des sichtbaren Spektrums härten lassen.

Diese Aufgabe wird durch polymerisierbare Zusammensetzungen gelöst, die
(a) 0,001 bis 5 Gew.-% Acylzinn-Verbindung(en) der allgemeinen Formel (I) als Photoinitiator,
(b) 10 bis 99,999 Gew.-% radikalisch polymerisierbares Bindemittel,
(c) 0 bis 85 Gew.-% Füllstoff und
(d) 0 bis 70 Gew.-% Additiv(e) enthalten, jeweils bezogen auf die Gesamtmasse der Zusammensetzung.

Die Zusammensetzungen enthalten als radikalische polymerisierbares Bindemittel mindestens ein mono- oder multifunktionelles (Meth)acrylat oder eine Mischung davon und als Photoinitiator mindestens eine Acylzinn-Verbindung gemäss der allgemeinen Formel (I) 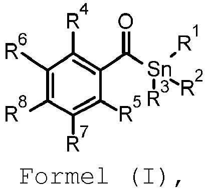 in der
- R¹,R²,R³: unabhängig voneinander jeweils eine Gruppe der Formel (II) 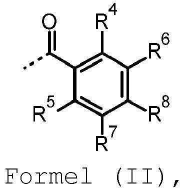 ein aromatischer C₆₋₃₀-Rest, der durch einen oder mehrere cyclische, verzweigte oder vorzugsweise lineare C₁₋₂₀-Alkyl-, C₁₋₂₀-Alkenyl-, C₁₋₂₀-Alkoxy-, C₁₋₂₀-Alkylthio- oder C₁₋₂₀-Alkenoxy-Reste substituiert sein kann, wobei die genannten Substituenten ihrerseits ein- oder mehrfach durch -O-, -S- oder -NR⁹- unterbrochen und/oder durch eine oder mehrere radikalisch polymerisationsfähige Gruppen und/oder Reste R¹⁰ substituiert sein können,
ein cyclischer, verzweigter oder vorzugsweise linearer C₁₋₂₀-Alkyl-, C₁₋₂₀-Alkenyl-, C₁₋₂₀-Alkoxy-, C₁₋₂₀-Alkylthio-, C₁₋₂₀-Alkenoxy- oder C₁₋₂₀-Acyloxy-Rest, der ein- oder mehr-fach durch -O-, -S- oder -NR⁹- unterbrochen und/oder durch eine oder mehrere radikalisch polymerisationsfähige Gruppen und/oder Reste R¹⁰ substituiert sein kann, oder ein Benzoyloxy-Rest,
-H, Trimethylsilyl, -OH, Halogen oder -CN sind,
wobei R¹ und R² auch zusammengefasst ein doppelt gebundenes Sauerstoff- oder Schwefelatom darstellen können oder zusammen mit dem Sn-Atom, an das sie gebunden sind, einen aliphatischen gesättigten oder ungesättigten Ring bilden können, der neben dem Sn-Atom 2 bis 6 Kohlenstoffatome und ggf. ein oder mehrere Sauerstoffatome enthält, wobei ein oder mehrere Kohlenstoffatome mit einem doppelt gebundenen Sauerstoffatom substituiert sein können und/oder der Ring mit einem aromatischen Ring anneliert sein kann,
- R⁴,R⁵,R⁶,R⁷,R⁸: unabhängig voneinander jeweils -H, ein cyclischer, verzweigter oder vorzugsweise linearer C₁₋₂₀-Alkyl-, C₁₋₂₀-Alkenyl-, C₁₋₂₀-Alkyloxy- oder C₁₋₂₀-Alkenoxy-Rest, der einoder mehrfach durch -O-, -S-, oder -NR⁹- unterbrochen und/oder durch eine oder mehrere radikalisch polymerisationsfähige Gruppen und/oder Reste R¹⁰ substituiert sein kann, -OR⁹, Halogen, -SR⁹, -N(R⁹)₂, -CF₃, -CN, -NO₂, -COOR⁹ oder -CONHR⁹ sind,
- R⁹: -H oder ein cyclischer, verzweigter oder vorzugsweise linearer C₁₋₂₀-Alkyl- oder C₁₋₂₀-Alkenyl-Rest ist und
- R¹⁰: -OH, -CₓF₂ₓ₊₁ mit x = 1 bis 20 oder -[Si(CH₃)₂]_{y}-CH₃ mit y = 1 bis 20 ist.

Wenn mehrere Reste eines Typs, z.B. mehrere Reste R⁴, R⁵, R⁶ usw., vorhanden sind, können diese verschieden oder vorzugsweise gleich sein. Bevorzugte radikalisch polymerisationsfähige Gruppen, die bei den Resten als Substituenten vorhanden sein können, sind Vinyl, Styryl, Acrylat (CH₂=CH-CO-O-), Methacrylat (CH₂=C(CH₃)-CO-O-), Acrylamid (CH₂=CH-CO-NR'- mit R' = H oder C₁-C₈-Alkyl), Methacrylamid (CH₂=C(CH₃)-CO-NH-), besonders bevorzugt (Meth)acrylat, Methacrylamid und/oder N-Alkylacrylamid. Vorzugsweise tragen die Reste 0 bis 3 und insbesondere 0 bis 1 radikalisch polymerisationsfähige Gruppen. Die polymerisationsfähigen Gruppen sind bei nichtzyklischen Resten vorzugsweise endständig angeordnet.

Durch die Formel (I) und die übrigen hierin gezeigten Formeln werden sämtliche stereoisomere Formen sowie Gemische verschiedener stereoisomerer Formen, wie z.B. Racemate, erfasst. Die Formeln erfassen nur solche Verbindungen, die mit der chemischen Valenzlehre vereinbar sind.

Der Hinweis, dass ein Rest durch ein Heteroatom wie O unterbrochen sein kann, ist so zu verstehen, dass die O-Atome in die Kohlenstoffkette oder den Kohlenstoffring des Restes eingeschoben werden, d.h. beidseitig von Kohlenstoffatomen begrenzt werden. Die Anzahl der Heteroatome ist daher um mindestens 1 kleiner als die Zahl der Kohlenstoffatome, und die Heteroatome können nicht endständig sein. Bei Kohlenwasserstoffresten, die Kohlenstoff- und Heteroatome enthalten, ist die Anzahl der Heteroatome ohne Berücksichtigung von Substituenten stets kleiner als die Zahl der Kohlenstoffatome.

Halogen (abgekürzt Hal) steht insbesondere für F, Cl, Br oder I, vorzugsweise für F, Cl und ganz besonders bevorzugt für Cl.

Bevorzugt sind insbesondere Acylzinn-Verbindungen gemäss der allgemeinen Formel (I), bei denen jeweils unabhängig voneinander
- R¹,R²,R³: unabhängig voneinander jeweils eine Gruppe der Formel (II), ein aromatischer C₆₋₁₅-Rest, der durch einen oder mehrere verzweigte oder vorzugsweise lineare C₁₋₁₂-Alkyl- oder C₁₋₁₂-Alkoxy-Reste substituiert sein kann, wobei die genannten Substituenten ihrerseits ein- oder mehrfach durch -O- unterbrochen und/oder durch eine oder mehrere radikalisch polymerisationsfähige Gruppen und/oder -OH substituiert sein können, ein verzweigter oder vorzugsweise linearer C₁₋₁₂-Alkyl-, C₁₋₁₂-Alkenyl-, C₁₋₁₂-Alkoxy-, C₁₋₁₂-Alkylthio- oder C₁₋₁₂-Acyloxy-Rest, der ein- oder mehrfach durch -O- unterbrochen und/oder durch eine oder mehrere radikalisch polymerisationsfähige Gruppen und/oder -OH substituiert sein kann, ein Benzoyloxy-Rest, Trimethylsilyl, -OH, Halogen oder -CN sind,
wobei R¹ und R² auch zusammengefasst ein doppelt gebundenes Sauerstoff- oder Schwefelatom darstellen können oder zusammen mit dem Sn-Atom, an das sie gebunden sind, einen aliphatischen gesättigten oder ungesättigten Ring bilden können, der neben dem Sn-Atom 2 bis 6, insbesondere 4, Kohlenstoffatome und ggf. ein oder mehrere, insbesondere 2, Sauerstoffatome enthält, wobei ein oder mehrere, insbesondere 2, Kohlenstoffatome mit einem doppelt gebundenen Sauerstoffatom substituiert sein können und/oder der Ring mit einem aromatischen, insbesondere sechsgliedrigen, Ring anneliert sein kann,
- R⁴,R⁵,R⁸: unabhängig voneinander jeweils -H, ein verzweigter oder vorzugsweise linearer C₁₋₁₂-Alkyl-, C₁₋₁₂-Alkenyl-, C₁₋₁₂-Alkyloxy- oder C₁₋₁₂-Alkenoxy-Rest sind, der ein- oder mehrfach durch -O- unterbrochen und/oder durch eine oder mehrere radikalisch polymerisationsfähige Gruppen und/oder -OH substituiert sein kann, -OR⁹, Halogen, -SR⁹, -N(R⁹)₂, -CF₃, -CN oder -NO₂ sind,
- R⁶,R⁷: unabhängig voneinander jeweils -H oder -F oder ein verzweigter oder vorzugsweise linearer C₁₋₁₂-Alkyl-, C₁₋₁₂-Alkenyl-, C₁₋₁₂-Alkyloxy- oder C₁₋₁₂-Alkenoxy-Rest, der einoder mehrfach durch -O- unterbrochen und/oder durch eine oder mehrere radikalisch polymerisationsfähige Gruppen und/oder -OH substituiert sein kann, sind und
- R⁹: -H oder Methyl ist.

Besonders bevorzugt sind Acylzinn-Verbindungen der Formel (I), bei denen jeweils unabhängig voneinander
- R¹,R²,R³: unabhängig voneinander jeweils eine Gruppe der Formel (II), Phenyl, Trimethylphenyl, ein verzweigter oder vorzugsweise linearer C₁₋₈-Alkyl-, C₁₋₁₂-Alkylthio- oder C₁₋₁₂-Acyloxy-Rest, ein Benzoyloxy-, Vinyl- oder MethacryloylRest, Trimethylsilyl, -OH, -Cl oder -CN sind,
wobei R¹ und R² auch zusammengefasst ein doppelt gebundenes Sauerstoff- oder Schwefelatom darstellen können oder zusammen mit dem Sn-Atom, an das sie gebunden sind, einen Dioxastannepin-Ring bilden können, wobei ein oder vorzugsweise zwei Kohlenstoffatome des Dioxastannepin-Rings mit einem doppelt gebundenen Sauerstoffatom substituiert sein können und/oder der Ring mit einem Benzol-Ring anneliert sein kann,
- R⁴,R⁵,R⁸: unabhängig voneinander jeweils -H, ein verzweigter oder vorzugsweise linearer C₁₋₈-Alkyl-Rest sind, der durch 1 bis 3 O-Atome unterbrochen und/oder durch Vinyl substituiert sein kann, -OR⁹, Halogen, -SR⁹, -N(R⁹)₂, -CF₃, -CN oder -NO₂ sind,
- R⁶,R⁷: unabhängig voneinander jeweils -H, -F oder ein verzweigter oder vorzugsweise linearer C₁₋₈-Alkyl-Rest sind, der durch 1 bis 3 O-Atome unterbrochen und/oder durch Vinyl substituiert sein kann, und
- R⁹: -H oder Methyl ist.

Ganz besonders bevorzugt sind Acylzinn-Verbindungen der Formel (I), bei denen jeweils unabhängig voneinander
- R¹,R²,R³: unabhängig voneinander jeweils eine Gruppe der Formel (II), Phenyl, ein linearer C₁-C₈-Alkylrest oder Trimethylsilyl sind,
- R⁴,R⁵,R⁸: unabhängig voneinander jeweils -H, Methyl, -OR⁹ oder -F sind,
- R⁶,R⁷: unabhängig voneinander jeweils -H oder -F sind und
- R⁹: -H oder Methyl ist.

Besonders bevorzugt sind Verbindungen, in denen alle Variablen jeweils eine der oben definierten bevorzugten Bedeutungen haben.

Die erfindungsgemäss verwendeten Acylzinn-Verbindungen der allgemeinen Formel (I) sind zum Teil bereits aus dem Stand der Technik bekannt. Die Synthese der Monoacylstannane kann zum Beispiel durch Lithiierung von Triorganozinnchlorid und anschliessende Umsetzung mit Säurechlorid erfolgen (vgl. G. J. D. Peddle, J. Organometal. Chem. 14 (1968) 139-147):

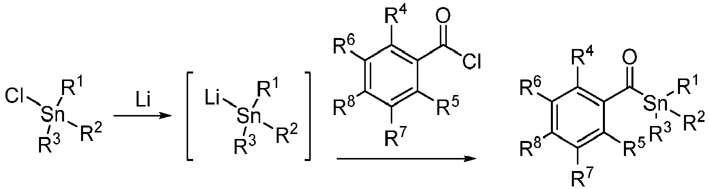

Konkretes Beispiel:

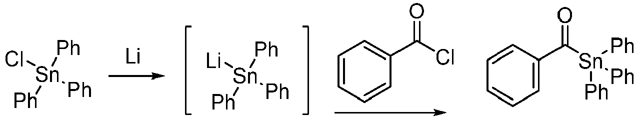

Eine weitere Möglichkeit zur Synthese von Acylzinn-Verbindungen ist eine Synthesevariante, bei der zunächst eine Sn-Grignard-Verbindung hergestellt wird und diese dann mit einem Aldehyd umgesetzt wird (vgl. J. P. Quintard, B. Elissondo, D. Mouko-Mpegna, J. Organomet. Chem. 251 (1983) 175-187):

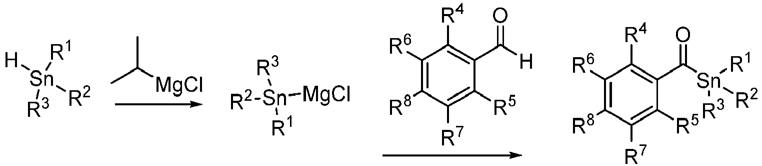

Konkretes Beispiel:

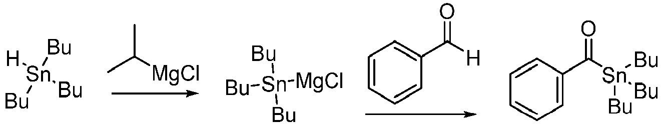

Bei einer weiteren Syntheseroute geht man von einer Hexaorganodizinn-Verbindung aus (vgl. T. N. Mitchell, K. Kwetkat, J. Organomet. Chem. 439 (1992) 127-138.):

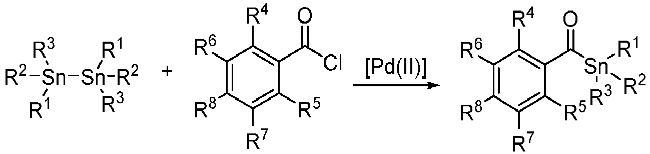

Konkretes Beispiel:

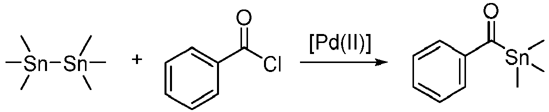

Weiterhin lassen sich Acylzinn-Verbindungen synthetisieren, indem ein Carbanion, welches aus 1,3-Dithianen erhalten wird, mit Zinnchloriden umgesetzt wird (vgl. A. G. Brook, J. M. Duff, P. F. Jones, N. R. Davis, J. Am. Chem. Soc. 89 (1967), 431-434):

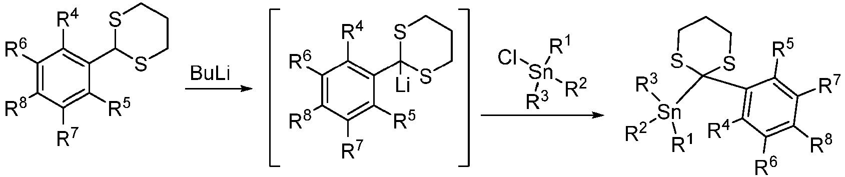

Die erhaltenen Dithiane können nach verschiedenen Methoden (vgl. A. G. Brook, J. M. Duff, P. F. Jones, N. R. Davis, J. Am. Chem. Soc. 89 (1967), 431-434) und J.-P. Bouillon, C. Portella, Eur. J. Org. Chem. 1999, 1571-1580), die dem Fachmann allgemein bekannt sind, zu den entsprechenden Ketonen hydrolysiert werden:

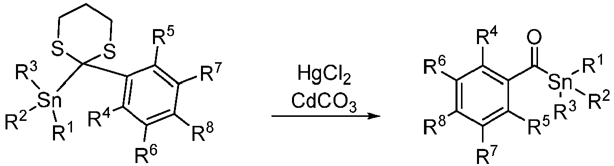

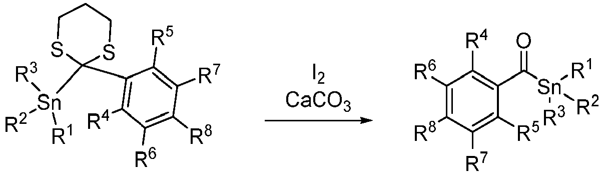

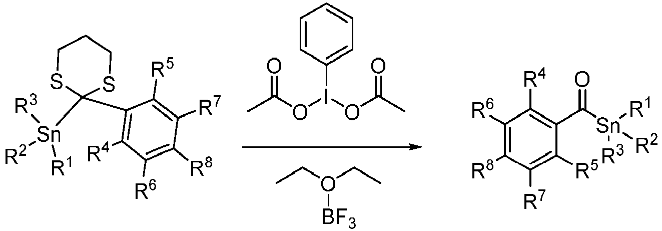

Analog zu den entsprechenden Germaniumverbindungen können Acylzinn-Verbindungen auch durch Oxidation von Hydroxygruppen erhalten werden (vgl. T. Nakamura, H. Yorimitsu, H. Shinokubo, K. Oshima, Tetrahedron 57 (2001) 9827-9836):

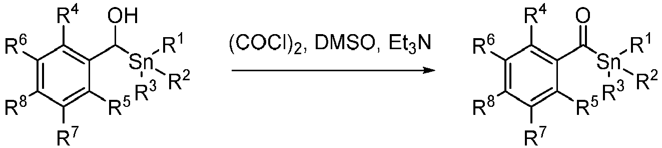

Konkrete Beispiele für besonders bevorzugte Verbindungen sind:

### Monoacylstannane:

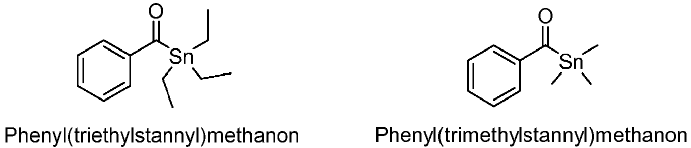

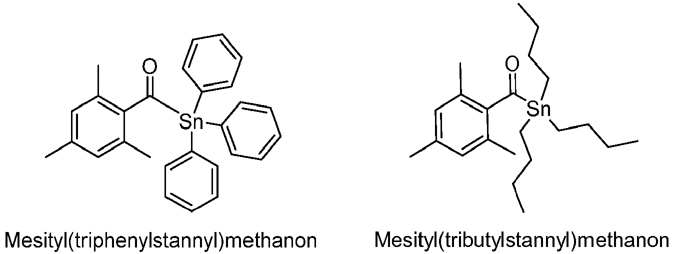

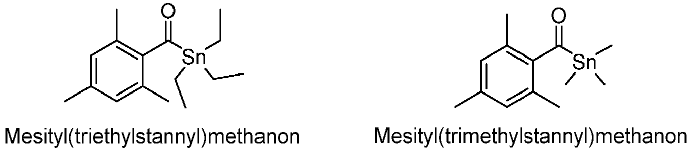

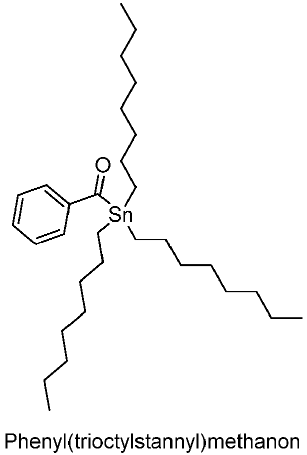

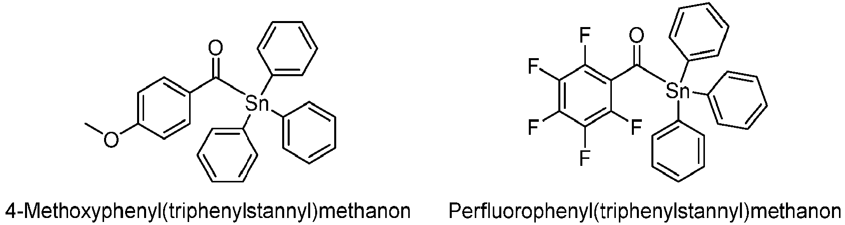

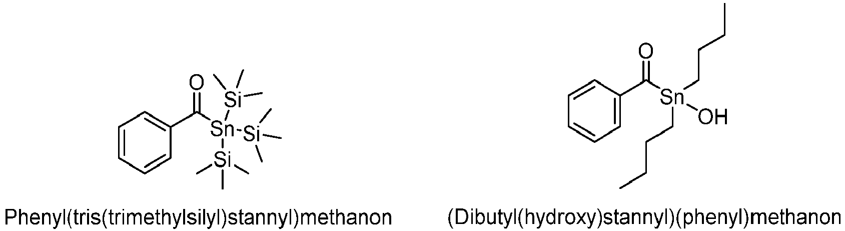

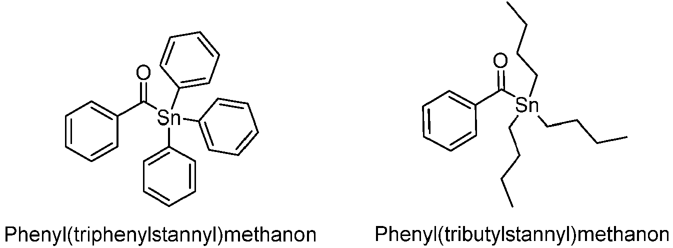

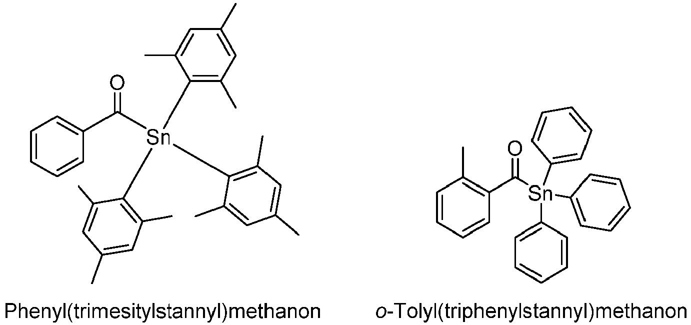

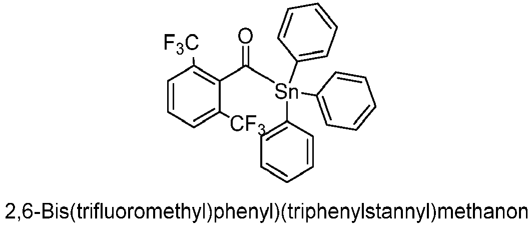

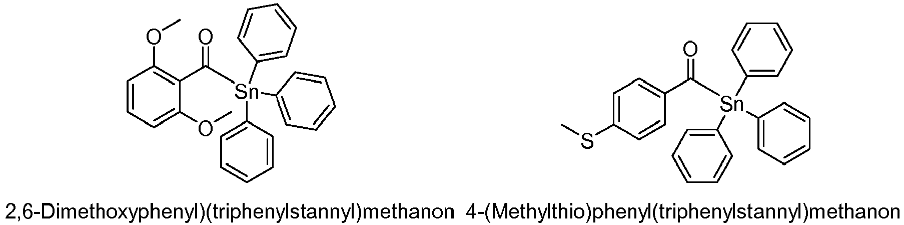

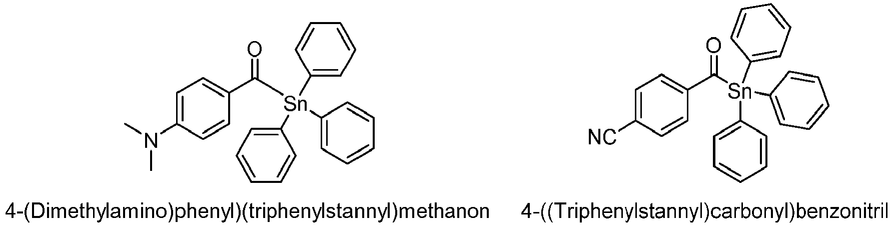

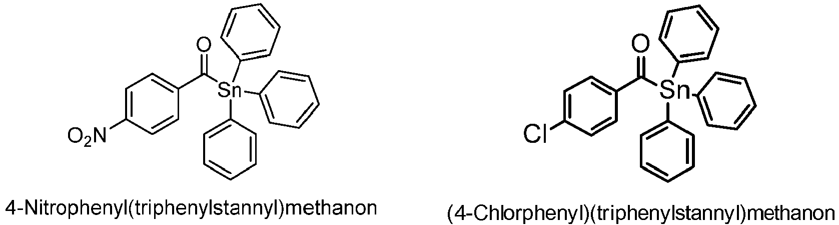

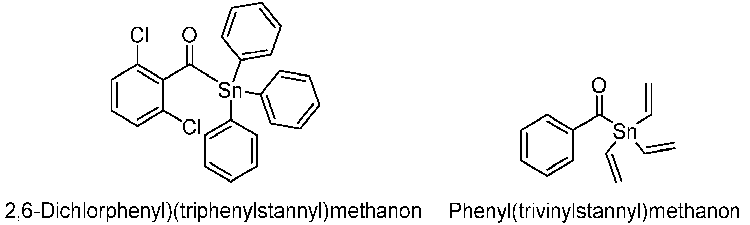

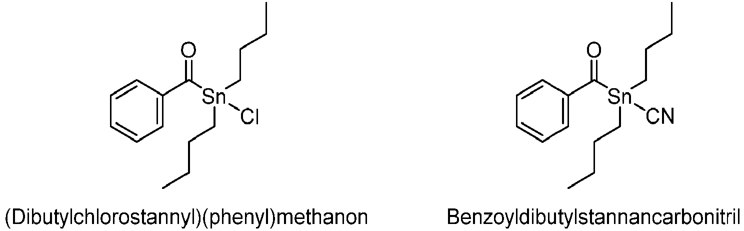

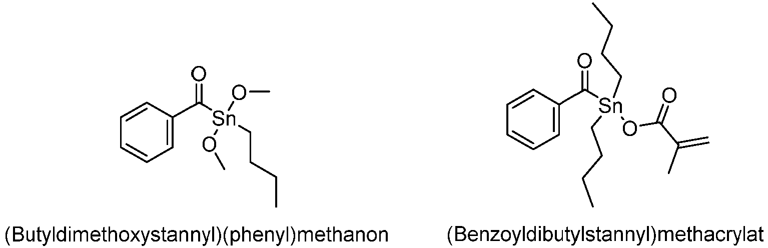

### Diacylstannane:

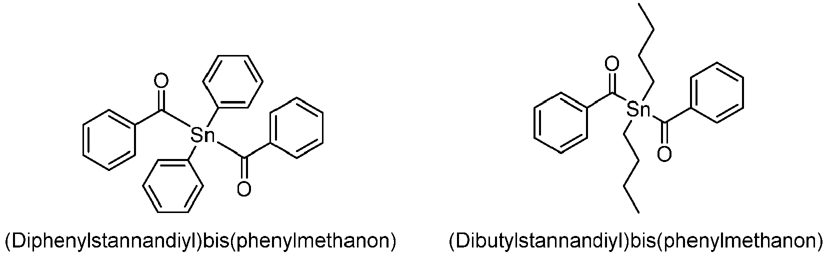

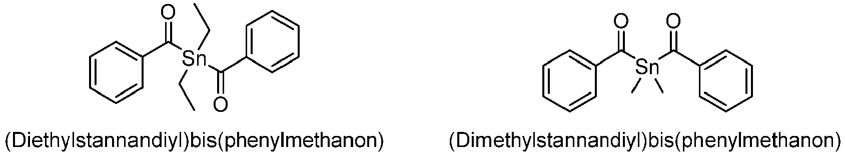

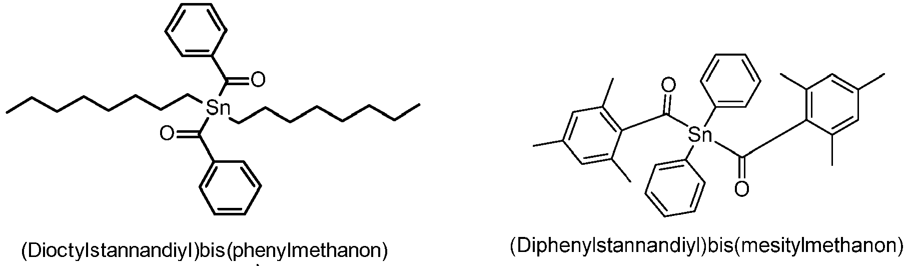

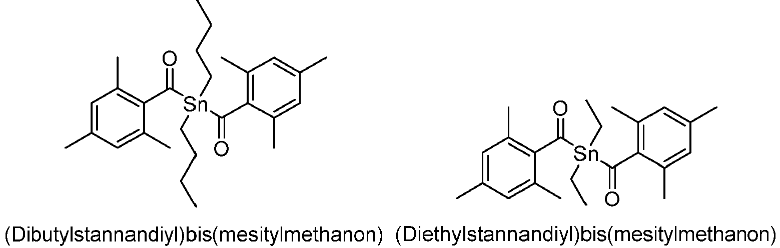

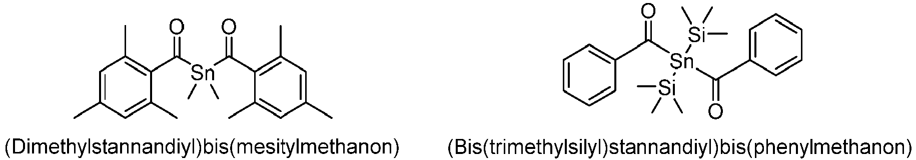

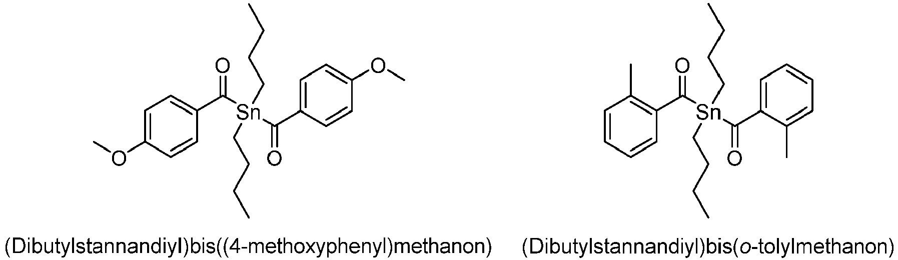

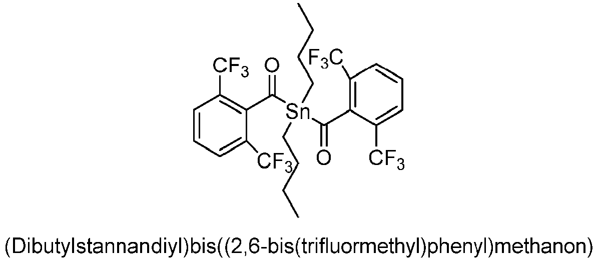

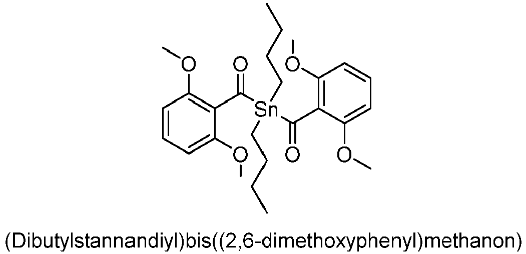

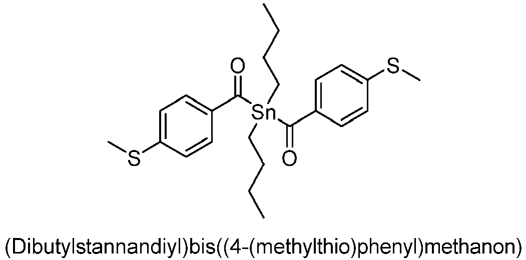

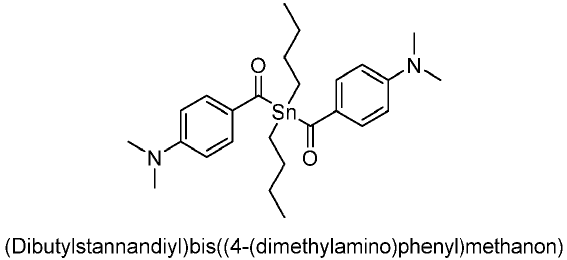

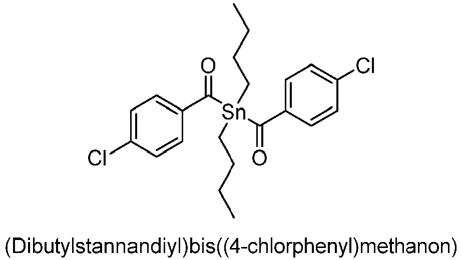

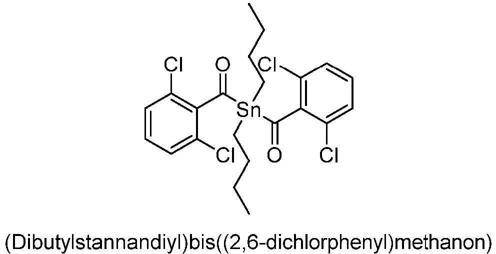

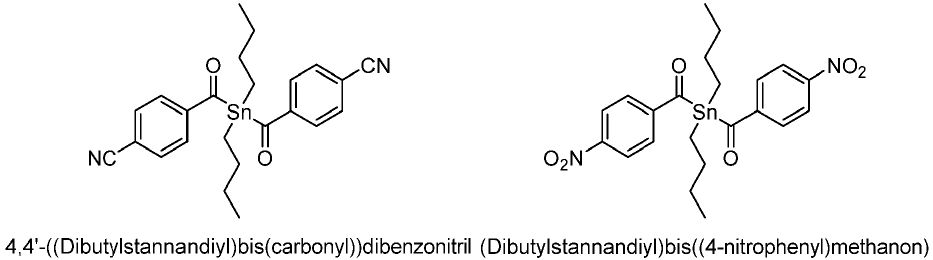

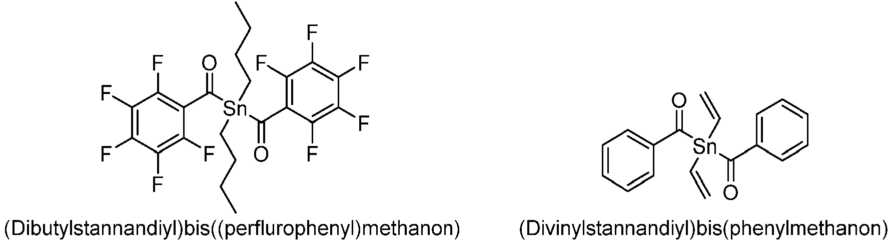

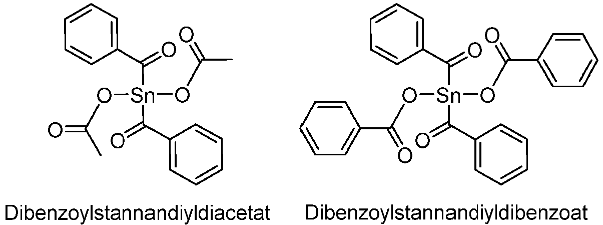

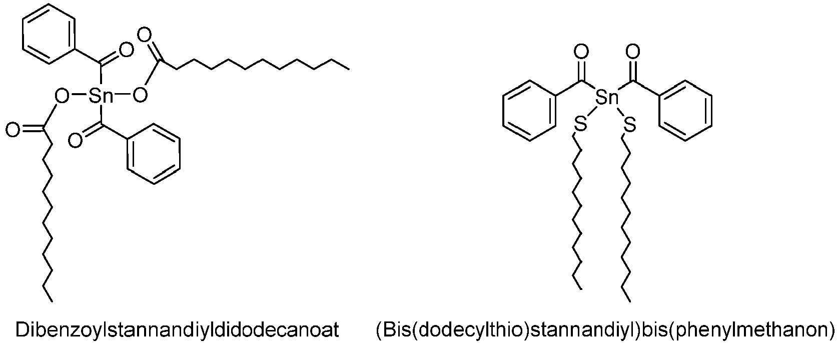

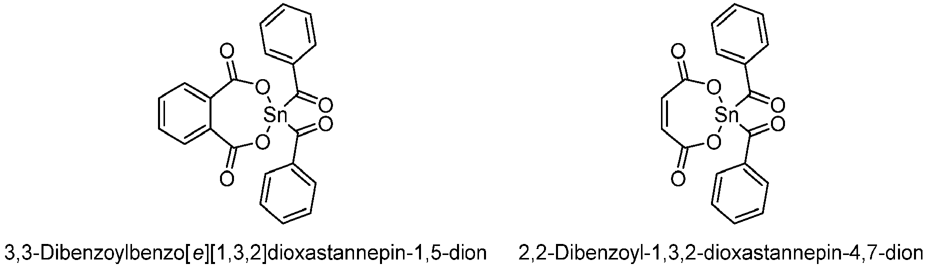

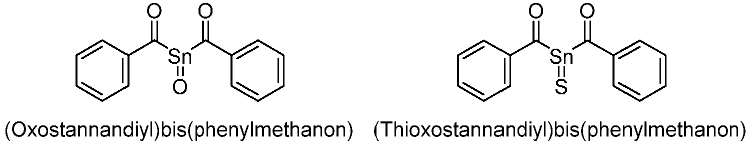

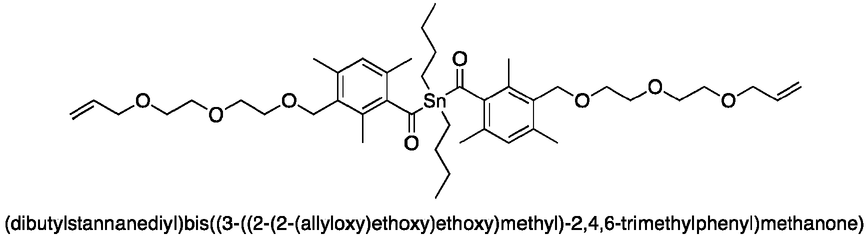

### Triacylstannane:

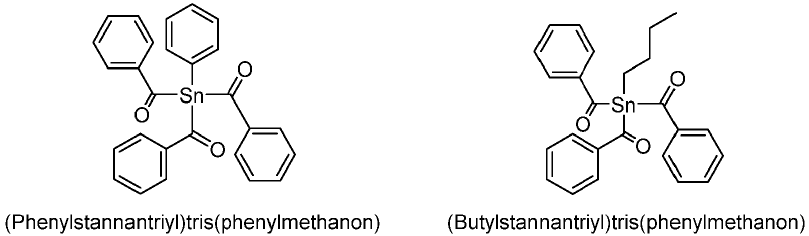

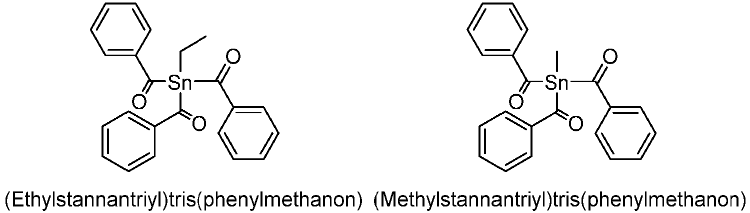

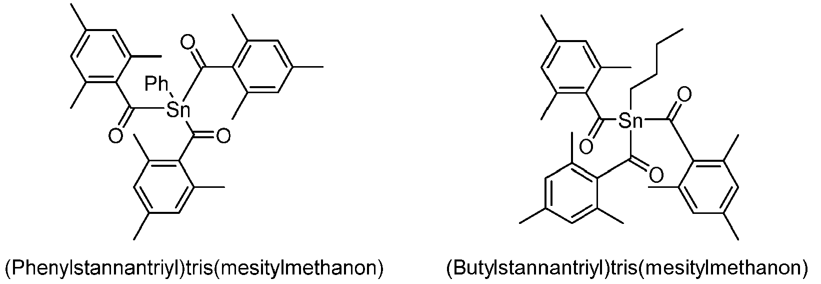

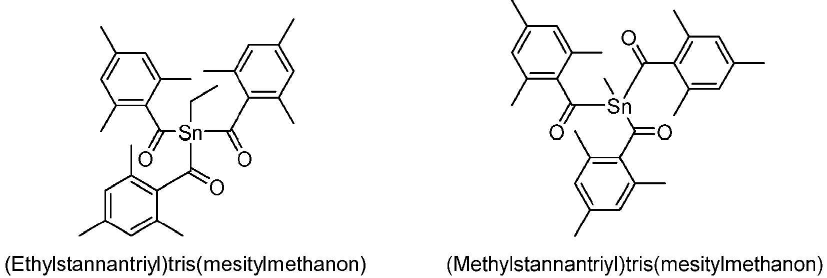

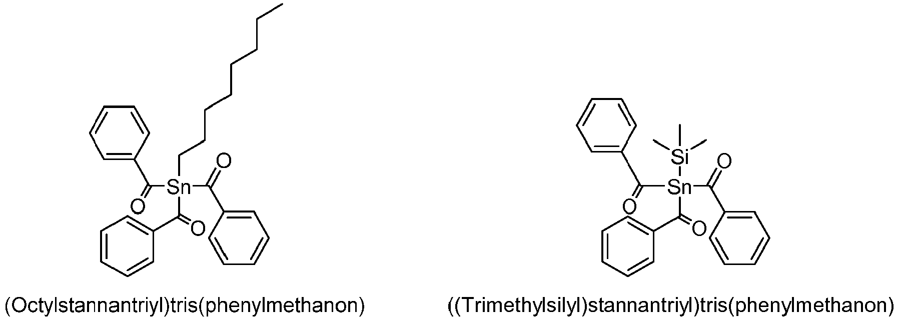

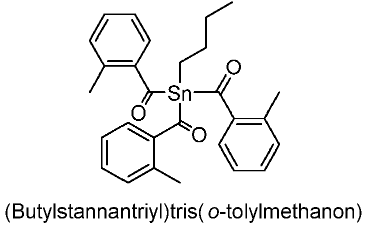

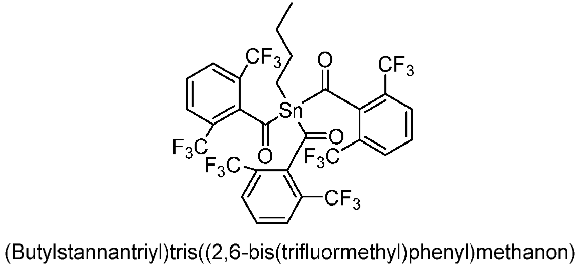

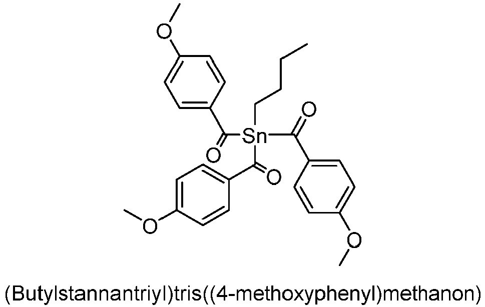

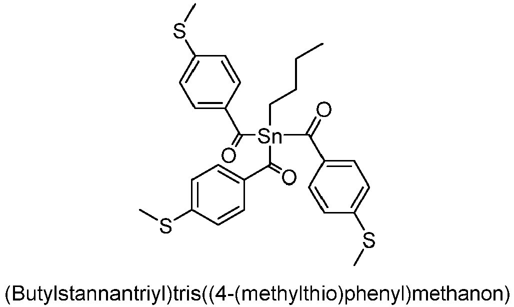

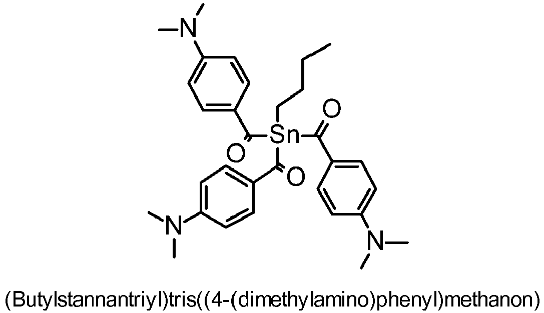

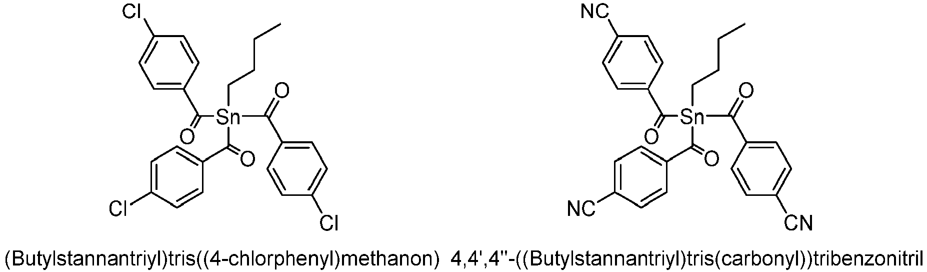

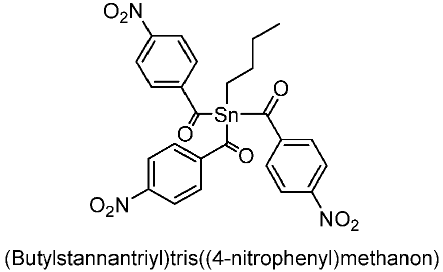

### Tetraacylstannane:

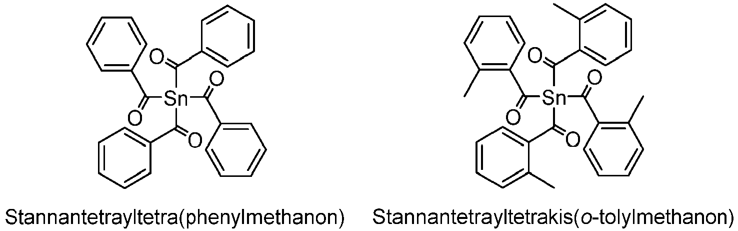

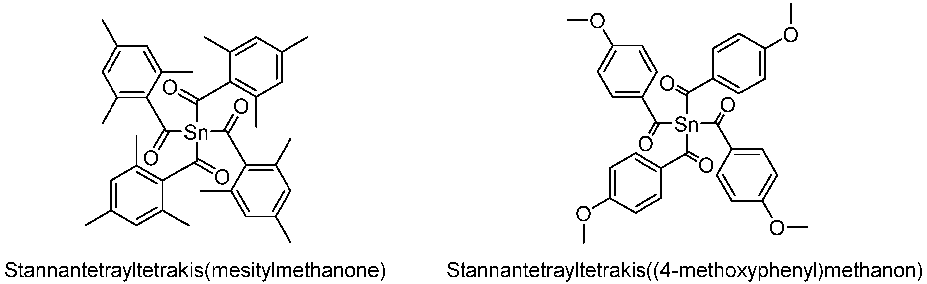

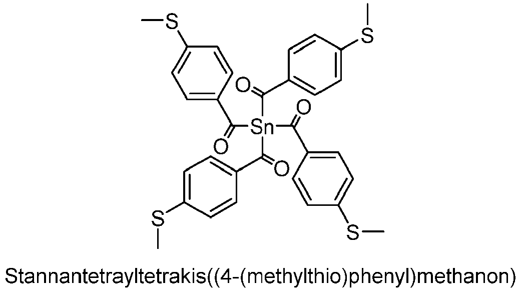

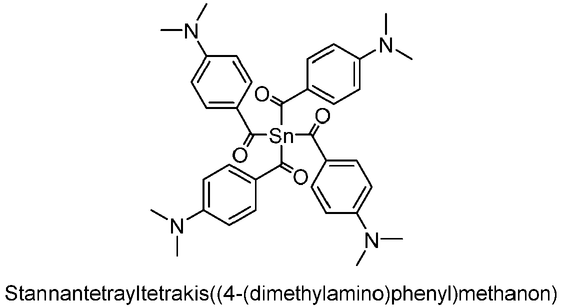

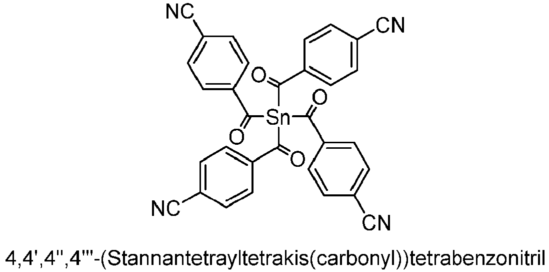

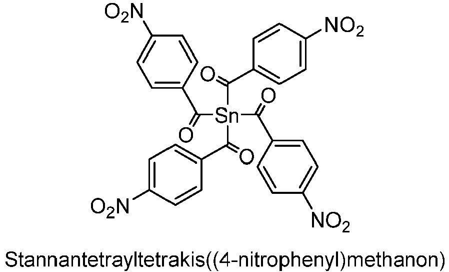

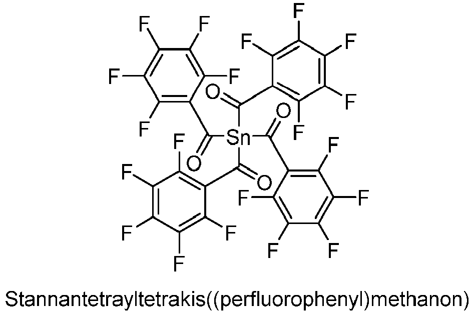

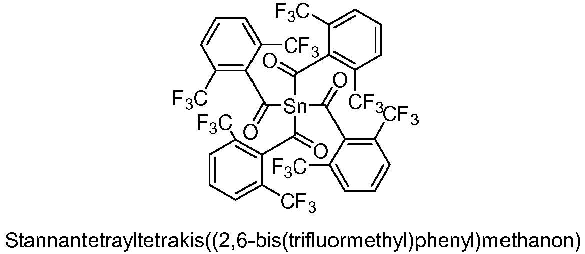

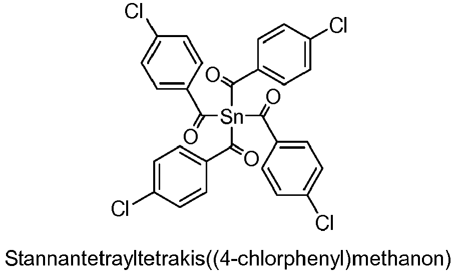

Es wurde überraschend gefunden, dass sich die Verbindungen der Formel (I) hervorragend als Photoinitiatoren für die Polymerisation und insbesondere für die radikalische Polymerisation eignen, besonders zur Initiierung von Zusammensetzungen auf Basis der nachfolgend definierten Monomere. Die Verbindungen der Formel (I) sind relativ einfach zugänglich und zeigen eine sehr gute polymerisationsauslösende Wirkung und ein ausgezeichnetes Bleachverhalten. Sie eignen sich darüber hinaus auch als Photoinitiatoren für andere Polyreaktionen wie z.B. die Polyaddition.

Die Verbindungen der Formel (I) eignen sich besonders für medizinische Anwendungen, beispielsweise zur Herstellung von Knochenzementen und von Kontaktlinsen, Intraokularlinsen oder anderen medizinischen Formteilen, wie z.B. von Gehörschalen, Knorpelimplantaten und künstlichen Gewebeteilen, und ganz besonders zur Herstellung von Dentalwerkstoffen wie Adhäsiven, Beschichtungen, Zementen und Kompositen. Die Acylzinn-Verbindungen der Formel (I) eignen sich darüber hinaus auch zur Herstellung von Werkstoffen für die Herstellung von Formteilen durch stereolithographische Verfahren.

Ausserdem eignen sich die Initiatoren der Formel (I) für eine Vielzahl von nicht-medizinischen Anwendungszwecken, wie zum Beispiel zur Herstellung von Formteilen, z.B. von Stäben, Platten, Scheiben oder Linsen etc., Druck- oder Anstrichfarben, Lacken, Adhäsiven, zur Herstellung von Druckplatten, integrierten Schaltkreisen, Photoresists, Lötmasken, Tinten für Farbdrucker, als Materialien für die holographische Datenspeicherung, zur Herstellung von nanodimensionierten mikroelektromechanischen Elementen, Lichtwellenleitern, Formteilen und zur optischen Herstellung von Informationsträgern. Ein wichtiges Anwendungsgebiet ist die Verwendung als Photoinitiator bei der stereolithographischen Herstellung von technischen Formteilen, z.B. von Präzisionsformteilen und von keramischen Grünkörpern.

Die erfindungsgemässen Zusammensetzungen enthalten bezogen auf die Gesamtmasse der Zusammensetzung vorzugsweise 0,01 bis 1,0 Gew.-% der Acylzinn-Verbindung der Formel (I).

Neben der Acylzinn-Verbindung der Formel (I) enthalten die Zusammensetzungen als radikalisch polymerisierbares Bindemittel mono- oder multifunktionelle (Meth)acrylate oder deren Mischungen. Unter monofunktionellen (Meth)acrylverbindungen werden Verbindungen mit einer, unter mehrfach funktionellen (Meth)acrylverbindungen Verbindungen mit zwei oder mehr, vorzugsweise 2 bis 3, polymerisierbaren Gruppen verstanden.

Diesbezügliche Beispiele sind Methyl-, Ethyl-, Hydroxyethyl-, Butyl-, Benzyl-, Tetrahydrofurfuryl- oder Isobornyl(meth)acrylat, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, sowie Gylcerindi- und -trimethacrylat, 1,4-Butandioldi(meth)acrylat, 1,10-Decandioldi(meth)acrylat oder 1,12-Dodecandioldi(meth)acrylat. Zusammensetzungen, die mindestens ein radikalisch polymerisierbares Monomer mit 2 oder mehr, vorzugsweise 2 bis 3, radikalisch polymerisierbaren Gruppen enthalten, sind besonders bevorzugt. Mehrfach funktionelle Monomere haben vernetzende Eigenschaften.

Als radikalisch polymerisierbare Bindemittel lassen sich auch hydrolysestabile Verdünnermonomere wie hydrolysestabile Mono-(meth)acrylate, z.B. Mesitylmethacrylat oder 2-(Alkoyxymethyl)-acrylsäuren, z.B. 2-(Ethoxymethyl)acrylsäure, 2-(Hydroxymethyl)-acrylsäure, N-mono- oder -disubstitiuierte Acrylamide, wie z.B. N-Ethylacrylamid, N,N-Dimethacrylamid, N-(2-Hydroxyethyl)acrylamid oder N-Methyl-N-(2-hydroxyethyl)acrylamid, bzw. N-monosubstituierte Methacrylamide, wie z.B. N-Ethylmethacrylamid oder N-(2-Hydroxyethyl)methacrylamid sowie ausserdem N-Vinylpyrrolidon oder Allylether einsetzen. Bevorzugte Beispiele für hydrolysestabile Vernetzermonomere sind Urethane aus 2-(Hydroxymethyl)-acrylsäure und Diisocyanten, wie 2,2,4-Trimethylhexamethylendiisocyanat oder Isophorondiisocyanat, vernetzende Pyrrolidone, wie z.B. 1,6-Bis(3-vinyl-2-pyrrolidonyl)-hexan, oder kommerziell zugängliche Bisacrylamide wie Methylen- oder Ethylenbisacrylamid, bzw. Bis(meth)acrylamide, wie z.B. N,N'-Diethyl-1,3-bis-(acrylamido)-propan, 1,3-Bis(methacrylamido)-propan, 1,4-Bis(acrylamido)-butan oder 1,4-Bis(acryloyl)-piperazin, die durch Umsetzung aus den entsprechenden Diaminen mit (Meth)acrylsäurechlorid synthetisiert werden können.

Weiterhin kann das radikalisch polymerisierbare Bindemittel auch bekannte schrumpfungsarme radikalisch ringöffnend polymerisierbare Monomere wie z.B. mono- oder multifunktionelle Vinylcyclopropane bzw. bicyclische Cyclopropanderivate (vgl. DE 196 16 183 C2 bzw. EP 1 413 569 A1) oder cyclische Allylsulfide (vgl. US 6,043,361 oder US 6,344,556)enthalten, die in Kombination mit den voranstehend aufgeführten Di(meth)acrylat-Vernetzern verwendet werden können. Geeignete ringöffnend polymerisierbare Monomere sind solche Vinylcyclopropane, wie 1,1-Di(ethoxycarbonyl)- oder 1,1-Di(methoxycarbonyl)-2-vinylcyclopropan oder die Ester der 1-Ethoxycarbonyl- oder 1-Methoxycarbonyl-2-vinylcyclopropancarbonsäure mit Ethylenglycol, 1,1,1-Trimethylolpropan, 1,4-Cyclohexandiol oder Resorcin. Geeignete bicyclische Cyclopropanderivate sind 2-(Bicyclo[3.1.0]hex-1-yl)acrylsäuremethyl- oder -ethylester oder deren Disubstitutionsprodukte in 3-Stellung wie (3,3-Bis(ethoxycarbonyl)bicyclo[3.1.0]-hex-1-yl)acrylsäuremethyl- oder -ethylester. Geeignete cyclische Allylsulfide sind die Additionsprodukte von 2-(Hydroxymethyl)-6-methylen-1,4-dithiepan oder 7-Hydroxy-3-methylen-1,5-dithiacylooctan mit 2,2,4-Trimethylhexamethylen-1,6-diisocyanat oder einem asymmetrischen Hexamethylendiisocyanat-Trimeren (z.B. Desmodur® VP LS 2294 der Bayer AG).

Geeignet sind auch Formulierungen die Vinylester, Vinylcarbonate oder Vinylcarbamate als radikalisch polymerisierbare Monomere enthalten. Ausserdem können als Monomere auch Styrol, Styrolderivate oder Divinylbenzol, ungesättigte Polyesterharze sowie Allylverbindungen oder radikalisch polymerisierbare Polysiloxane, die aus geeigneten Methacrylsilanen, wie z.B. 3-(Methacryloyloxy)propyltrimethoxysilan, hergestellt werden können und z.B. in der DE 199 03 177 C2 beschrieben sind, eingesetzt werden.

Weiterhin lassen sich als radikalisch polymerisierbare Bindemittel auch Mischungen der voranstehend genannten Monomere mit radikalisch polymerisierbaren, säuregruppenhaltigen Monomeren verwenden, die auch als Haftmonomere bezeichnet werden. Bevorzugte säuregruppenhaltige Monomere sind polymerisationsfähige Carbonsäuren, wie Maleinsäure, Acrylsäure, Methacrylsäure, 2-(Hydroxymethyl)acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin oder 4-Vinylbenzoesäure.

Als Haftmonomere eignen sich auch radikalisch polymerisierbare Phosphonsäuremonomere, insbesondere Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure, 2-[4-(Dihydroxyphosphoryl)-2-oxa-butyl]-acrylsäure oder 2-[2-Dihydroxyphosphoryl)-ethoxymethyl]-acrylsäure-ethyl- oder 2,4,6-trimethylphenylester.

Zudem eignen sich als Haftmonomere acide polymerisationsfähige Phosphorsäureester, insbesondere 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, Dipentaerythritol-pentamethacryloyloxyphosphat, 10-Methacryloyloxydecyldihydrogenphosphat, Dipentaerythritolpentamethacryloyloxyphosphat, Phosphorsäuremono-(1-acryloyl-piperidin-4-yl)-ester, 6-(Methacrylamido)hexyldihydrogenphosphat und 1,3-Bis-(N-acryloyl-N-propylamino)-propan-2-yl-dihydrogenphosphat.

Darüber hinaus eignen sich polymerisationsfähige Sulfonsäuren als Haftmonomere, insbesondere Vinylsulfonsäure, 4-Vinylphenylsulfonsäure oder 3-(Methacrylamido)propylsulfonsäure.

Neben Acylzinn-Verbindungen der allgemeinen Formel (I) können die erfindungsgemässen Zusammensetzungen vorteilhaft zusätzlich auch bekannte Photoinitiatoren (vgl. J.P. Fouassier, J.F. Rabek (Hrsg.), Radiation Curing in Polymer Science and Technology, Vol. II, Elsevier Applied Science, London und New York 1993) für den UV- oder sichtbaren Bereich, wie z.B. Benzoinether, Dialkylbenzilketale, Dialkoxyacetophenone, Acyl- oder Bisacylphosphinoxide, α-Diketone wie 9,10-Phenanthrenchinon, Diacetyl, Furil, Anisil, 4,4'-Dichlorbenzil und 4,4'-Dialkoxybenzil und Campherchinon enthalten. Gut geeignet sind Kombinationen mit Norrish-Typ-I-Photoinitiatoren, vor allem Acyl- oder Bisacylphosphinoxiden, wie beispielsweise die kommerziell erhältlichen Verbindungen 2,4,6-Trimethylbenzoyldiphenylphosphinoxid und Bis-(2,4,6-trimethylbenzoyl)phenylphosphinoxid, und besonders geeignet sind Monoacyltrialkyl-, Diacyldialkylgermanium-, Triacylalkyl- sowie Tetraacylgermanium-Verbindungen, wie z.B. Benzoyltrimethylgermanium, Dibenzoyldiethylgermanium oder Bis(4-methoxybenzoyl)diethylgermanium sowie Tetrabenzoylgermanium. Dabei lassen sich auch Mischungen verschiedener Photoinitiatoren einsetzen. Es können auch Initiatorkombinationen der Acylzinn-Verbindungen der allgemeinen Formel (I) eingesetzt werden, die zusätzlich aromatische Diaryliodonium- oder Triarylsulfoniumsalzen enthalten, beispielsweise die kommerziell zugänglichen Verbindungen 4-Octyloxyphenyl-phenyl-iodoniumhexafluoroantimonat oder Isopropylphenylmethylphenyl-iodoniumtetrakis(pentafluorphenyl)borat.

Ausserdem können die erfindungsgemässen Zusammensetzungen neben den Acylzinn-Verbindungen der allgemeinen Formel (I) zur dualen Härtung auch Azoverbindungen, wie 2,2'-Azobis(isobutyronitril) (AIBN) oder Azobis-(4-cyanovaleriansäure), oder Peroxide, wie Dibenzoylperoxid, Dilauroylperoxid, tert-Butylperoctoat, tert-Butylperbenzoat oder Di-(tert-butyl)-peroxid enthalten. Zur Beschleunigung der Initiierung mittels Peroxiden lassen sich auch Kombinationen mit aromatischen Aminen einsetzen. Redoxsysteme, die sich bereits bewährt haben, sind: Kombinationen aus Benzoylperoxid mit Aminen, wie N,N-Dimethyl-p-toluidin, N,N-Dihydroxyethyl-p-toluidin, p-Dimethylaminobenzoesäureethylester oder strukturverwandte Systeme. Darüber hinaus sind auch Redoxsysteme bestehend aus Peroxiden und solchen Reduktionsmitteln, wie z.B. Ascorbinsäure, Barbiturate oder Sulfinsäuren oder Kombinationen von Hydroperoxiden mit Reduktionsmitteln und katalytischen Metallionen, wie z.B. eine Mischung von Cumolhydroperoxid, einem Thioharnstoffderivat und Kupfer(II)-acetylacetonat, für die duale Aushärtung geeignet.

Erfindungsgemäss sind Zusammensetzungen bevorzugt, die einen oder mehrere Füllstoffe, vorzugsweise organische oder anorganische partikuläre Füllstoffe enthalten. Vorzugsweise werden als Füllstoffe partikuläre Materialien mit einer mittleren Partikelgrösse von 1 nm bis 10 µm, vorzugsweise von 5 nm bis 5 µm verwendet. Der Begriff "mittlere Partikelgrösse" bezieht sich hier jeweils auf das Volumenmittel.

Bevorzugte anorganische partikulären Füllstoffe, insbesondere für den dentalen Bereich, sind amorphe kugelförmige nanopartikuläre Füllstoffe auf der Basis von Oxiden, wie pyrogene Kieselsäure oder Fällungskieselsäure, ZrO₂ und TiO₂ oder Mischoxide aus SiO₂, ZrO₂ und/oder TiO₂ mit einer mittleren Partikelgrösse von 10 bis 200 nm, Minifüllstoffe, wie Quarz-, Glaskeramik- oder Glaspulver mit einer mittleren Partikelgrösse von 0,2 bis 5 µm sowie röntgenopake Füllstoffe, wie Ytterbiumtrifluorid oder nanopartikuläres Tantal(V)-oxid bzw. Bariumsulfat. Darüber hinaus können auch faserförmige Füllstoffe wie Nanofasern, Glasfasern, Polyamid- oder Kohlenstoff-Fasern eingesetzt werden.

Für nichtdentale Anwendungen kommen neben den oben genannten Materialien ausserdem Homo- und/oder Copolymere, vorzugsweise Poly((meth)acrylat)e, Vinylpolymere, vorzugsweise Polystyrol oder Polyvinylacetat, oder Kondensationspolymere, vorzugsweise Polyester, als Füllstoffe in Betracht. Diese Füllstoffe werden vorzugsweise als Pulver mit einer mittleren Partikelgrösse zwischen 0,5 und 100 µm eingesetzt. Sie sind zum Teil im Monomer löslich. Weiterhin können für nichtdentale Anwendungen auch anorganische Füllstoffe wie z.B. CaCO₃, Talkum, TiO₂, CaSO₄, Silikate, Glas, Kohlenstoffpulver oder -fasern oder Metallpulver auf Basis von Al, Zn, Cu oder Ni eingesetzt werden.

Ausserdem können die erfindungsgemässen Zusammensetzungen im Bedarfsfalle weitere Additive, wie z.B. Stabilisatoren, UV-Absorber, Farbstoffe oder Pigmente, sowie Lösungsmittel, wie z.B. Wasser, Ethanol, Aceton oder Ethylacetat, oder Gleitmittel enthalten.

Die erfindungsgemässen Werkstoffe enthalten vorzugsweise:
(a) 0,001 bis 5 Gew.-% Acylzinn-Verbindung(en) der allgemeinen Formel (I),
(b) 10 bis 99,999 Gew.-% radikalisch polymerisierbares Bindemittel,
(c) 0 bis 85 Gew.-% Füllstoff und
(d) 0 bis 70 Gew.-% Additiv(e).

Alle Prozentangaben beziehen sich, wenn nicht anders angegeben, auf die Gesamtmasse des Materials.

Werkstoffe, die sich besonders als dentale Zemente eignen, enthalten bevorzugt:
(a) 0,001 bis 5 Gew.-% Acylzinn-Verbindung(en) der allgemeinen Formel (I),
(b) 10 bis 50 Gew.-% radikalisch polymerisierbares Bindemittel,
(c) 40 bis 70 Gew.-% Füllstoff und
(d) 0 bis 5 Gew.-% Additiv(e).

Werkstoffe, die sich besonders als dentale Komposite eignen, enthalten bevorzugt:
(a) 0,001 bis 5 Gew.-% Acylzinn-Verbindung(en) der allgemeinen Formel (I),
(b) 10 bis 40 Gew.-% radikalisch polymerisierbares Bindemittel,
(c) 50 bis 85 Gew.-% Füllstoff und
(d) 0 bis 5 Gew.-% Additiv(e).

Werkstoffe, die sich besonders als dentale Beschichtungsmaterialien eignen, enthalten bevorzugt:
(a) 0,001 bis 5 Gew.-% Acylzinn-Verbindung(en) der allgemeinen Formel (I),
(b) 10 bis 99,989 Gew.-% radikalisch polymerisierbares Bindemittel,
(c) 0 bis 20 Gew.-% nanopartikuläre Füllstoffe,
(d) 0,01 bis 2 Gew.-% Additiv(e) und
(e) 0 bis 70 Gew.-% Lösungsmittel.

Werkstoffe, die sich besonders als Beschichtungen eignen, enthalten bevorzugt:
(a) 0,001 bis 5 Gew.-% Acylzinn-Verbindung(en) der allgemeinen Formel (I),
(b) 20 bis 99 Gew.-% radikalisch polymerisierbares Bindemittel,
(c) 0 bis 20 Gew.-% Farbstoff(e) und/oder Pigment(e) und
(d) 0 bis 10 Gew.-% weitere Additiv(e).

Werkstoffe, die sich besonders als Druckfarben eignen, enthalten bevorzugt:
(a) 0,001 bis 5 Gew.-% Acylzinn-Verbindung(en) der allgemeinen Formel (I),
(b) 20 bis 95 Gew.-% radikalisch polymerisierbares Bindemittel,
(c) 1 bis 20 Gew.-% Farbstoff(e) und/oder Pigment(e) und
(d) 0 bis 10 Gew.-% weitere Additiv(e).

Die erfindungsgemässen Zusammensetzungen eignen sich zur Herstellung von Photopolymerisaten sowie als Komposite, Zemente, Beschichtungsmaterialien, Primer oder Adhäsive. Sie eignen sich besonders für Anwendungen im medizinischen Bereich, vor allem als Dentalwerkstoffe, wie Füllungskomposite, Befestigungszemente, Adhäsive, Prothesenmaterialien, Verblendmaterialien, sowie zur Herstellung von Kronen, Inlays oder von Beschichtungen.

Die Dentalwerkstoffe eignen sich zur intraoralen Anwendung durch den Zahnarzt zur Restauration geschädigter Zähne, d.h. zur therapeutischen Anwendung, z.B. als dentale Zemente, Füllungskomposite und Verblendmaterialien. Sie können aber auch extraoral eingesetzt werden, beispielsweise bei der Herstellung oder Reparatur von Dentalrestaurationen, wie Prothesen, künstlichen Zähnen, Inlays, Onlays, Kronen und Brücken.

Ferner eignen sich die erfindungsgemässen Materialien zur medizinischen Anwendung in der Chirurgie, z.B. in der Geweberegeneration, zur Herstellung von Gehörhilfen oder in der Augenheilkunde zur Herstellung von Intraokularlinsen oder Kontaktlinsen.

Bei technischen Anwendungen lassen sich die erfindungsgemässen Zusammensetzungen in der Stereolithographie oder im 3D-Druck zur Herstellung von Formkörpern, Prototypen oder Grünkörpern, im Beschichtungsbereich oder auch in der Mikroelektronik z.B. in der Photoresist-Technologie oder der Nano-Imprint-Lithographie einsetzen. Eine besonders bevorzugte Anwendung der erfindungsgemässen Materialien liegt dabei im Bereich des 3D-Drucks von Keramik- oder Metallpulvern über Lithographie-basierte Verfahren. Hierbei stellt das erfindungsgemäss hergestellte Photopolymer die Opferstruktur beim Sinterprozess dar. Aufgrund des relativ langwelligen Absorptionsmaximums der erfindungsgemässen Verbindungen der Formel (I) können dabei auch Keramiken, wie z.B. Zirkonoxide, Carbide und Nitride, z.B. auf Siliziumbasis, und sogar Metallpulver, z.B. Edelstahl oder Werkzeugstähle, gedruckt werden.

Ausserdem eignen sich die erfindungsgemässen Zusammensetzungen und die daraus hergestellten Polymerisate auch als Lacke oder Beschichtungen auf diversen Oberflächen, z.B. als dekorative Beschichtungen und Schutzschichten auf Holz, Papier, Karton und insbesondere Kunststoff, Keramik oder Metall. Dabei bietet das relativ langwellige Absorptionsmaximum der erfindungsgemässen Verbindungen der Formel (I) besondere Vorteile insbesondere für die Durchhärtungstiefe von pigmentierten oder gefüllten Systemen etwa im Bereich der Inkjet-Verfahrens. Ferner können die erfindungsgemässen Zusammensetzungen und die daraus hergestellten Polymerisate auch als Klebstoffe zum Verbinden unterschiedlichster Werkstoffe oder auch zur Herstellung von Formkörpern durch Giessen, Pressen, Rapid Prototyping oder 3D-Druck dienen.

Die Erfindung wird im Folgenden anhand von Beispielen näher erläutert.

### Beispiel 1:

### Synthese von Benzoyltriphenylzinn (BtPhSn)

Unter Zuhilfenahme einer Glove Box wurden 1 Äq. (1,69 g) Triphenylzinnchlorid und 3 Äq. (0,09 g) Lithiumfolie in separate Schlenkkolben eingewogen und danach zu jedem dieser Kolben 10 ml wasserfreies THF hinzugefügt. Die Reaktionsgefässe wurden an eine Inertgashahnleiste im Orangelichtlabor angeschlossen und anschliessend wurden 0,05 Äq. (0,03 g) Naphthalin (als Katalysator) zur Lithiumsuspension zugegeben. Die Suspension wurde für 20 Minuten bei Raumtemperatur (RT) in ein Ultraschallbad gestellt und danach für weitere 30 Minuten intensiv gerührt, woraufhin sich die Suspension rasch dunkel färbte. Die Triphenylzinnchlorid-Lösung wurde dann mittels Spritze und Septum zur Lithiumsuspension bei RT über einen Zeitraum von 20 Minuten zugetropft, wodurch sich die Reaktionsmischung kurzzeitig wieder entfärbte. Es wurde für 4 h bei RT gerührt und ein weiterer Schlenkkolben mit 10 ml einer Lösung von 1,06 Äq. (0,54 ml) Benzoylchlorid in trockenem THF vorbereitet. Beide Lösungen wurden mittels einer Aceton/N₂-Mischung auf -78 °C gekühlt und die erste Reaktionsmischung dann über einen Zeitraum von 20 Minuten zur Benzoylchlorid-Lösung zugetropft. Nach beendeter Zugabe wurde das Kühlbad entfernt und die Mischung unter Lichtausschluss für 18 h bei RT gerührt. Das Lösungsmittel wurde im Feinvakuum abgezogen. Das verbleibende Substanzgemisch wurde in 20 ml wasserfreiem n-Pentan aufgenommen und dann unter inerten Bedingungen filtriert. Es wurde dann zweimal mit je 10 ml wasserfreiem n-Pentan nachgewaschen und das Lösungsmittel dann unter Lichtausschluss vom Filtrat abgezogen. Die erhaltenen intensiv gelben Kristalle wurden dann im Hochvakuum getrocknet und unter Lichtausschluss und unter Argon im Kühlschrank (4°C) gelagert. Das erhaltene Produkt (1,25 g, 63% der Theorie, Schmelzpunkt: 58 °C) weist bereits ohne Aufreinigungsschritt eine sehr gute Reinheit auf, was durch GC-MS, HPLC und NMR-Spektroskopie bestätigt werden konnte.
GC-MS (CH₂Cl₂) : m/z (rel.) : 455 (M, 4%), 379 (6%), 351 (100%), 197 (44%), 120 (10%), 77 (10%).
¹H-NMR: δ_{H} (400 MHz, CDCl₃) : 7,82-7,28 (20H, m, Ar-H) .
¹³C-NMR: δ_{C} (100MHz, CDCl₃): 161,35 (**C**=O); 141,56; 137,24; 136,14; 135,11; 132,64; 128,31; 128,04; 127,82; 127,73 (**C**ₐᵣₒₘ). ¹¹⁹Sn-NMR: δ_{Sn} (CDCl₃) : -217,74.

Das Absorptionsspektrum einer Acetonitrillösung von **BtPhSn** mit einer Konzentration von 1,0 mmol/l im Vergleich zu Benzoyltrimethylgermanium ist in Figur 1 dargestellt. Das Absorptionsmaximum von **BtPhSn** liegt bei 430 nm (Benzoyltrimethylgermanium: 411 nm) und der Extinktionskoeffizient beträgt 309 l/cm·mol (Benzoyltrimethylgermanium: 146 l/cm·mol). Ausserdem wird bei **BtPhSn** erst bei Wellenlängen oberhalb von 500 nm keine signifikante Absorption mehr beobachtet, während dies bei Benzoyltrimethylgermanium bereits ab etwa 465 nm der Fall ist. Der Vergleich zeigt, dass das Benzoylchromophor der Sn-Verbindung stärker und langwelliger im Vergleich zur Ge-Verbindung absorbiert, was für die Anwendung als Photoinitiator vorteilhaft ist.

### Beispiel 2:

### Photolyse einer Lösung von BtPhSn

Eine Acetonitrillösung von **BtPhSn** aus Beispiel 1 mit einer Konzentration von 1,0 mmol/l und einer MMA-Konzentration von 0,5 mol/L wurde in einer 1-cm-Küvette mit der Polywave LED Bluephase 20 i (Ivoclar Vivadent AG) mit einem halogenähnlichen Breitbandspektrum von 385-515 nm bestrahlt und das Absorptionsspektrum in Abhängigkeit von der Bestrahlungszeit aufgenommen. Die Ergebnisse sind im Vergleich zu Benzoyltrimethylgermanium **(BtMGe)** in Figur 2 dargestellt. Die Ergebnisse belegen, dass der Sn-Photoinitiator **BtPhSn** bei Bestrahlung deutlich schneller ausbleicht als ein analoger Ge-Photoinitiator **BtMGe.**

### Beispiel 3:

### Herstellung von lichthärtbaren Kompositen unter Verwendung von Benzoyltriphenylzinn (BtPhSn) aus Beispiel 1

Aus einer Mischung (Angaben in Masse-%) der Dimethacrylate UDMA (Additionsprodukt aus 2-Hydroxyethylmethacrylat und 2,2,4-Trimethylhexamethylendiisocyanat) bzw. TEGDMA (Triethylenglycol) bzw. Bis-GMA (Additionsprodukt aus Methacrylsäure und Bisphenol-A-diglycidylether) und dem Sn-Photoinitiator **BtPhSn** aus Beispiel 1 sowie Füllstoffen: OX-50, sil. (silanisierte pyrogenen Kieselsäure, Degussa), YbF₃ (Ytterbiumtrifluorid, Sukgyung, Südkorea), SiO₂-ZrO₂-Mischoixid, sil. (silanisiertes SiO₂-ZrO₂-Mischoxid, Transparent Materials, USA) und Glasfüller GM 27884, sil. (silanisierter Glasfüller GM 27884, 1,0 µm, Schott) (Tabelle 1) wurden die lichthärtende Komposite **C1** (zementähnliche Konsistenz) und **C2** (Füllungskomposit) mittels eines Walzenstuhles "Exakt" (Exakt Apparatebau, Norderstedt) hergestellt.

**Tabelle 1: Zusammensetzung der Komposite C1 und C2**

| **Komponente/Harz [Gew.-%]** | **C1** | **C2** |
|---|---|---|
| **BtPhSn** | 0,82 | 0,10 |
| UDMA | 31,34 | 7,49 |
| TEGDMA | 7,86 | 4,05 |
| Bis-GMA | - | 8,46 |
| OX-50, sil. | 41,21 | 1,02 |
| YbF₃ | 18,77 | 17,21 |
| SiO₂-ZrO₂-Mischoixid, sil. | - | 10,12 |
| Glasfüller GM 27884, sil. | - | 51,55 |

Von den Materialien wurden entsprechende Prüfkörper präpariert, die 2 mal 3 Minuten mit einer dentalen Lichtquelle (Spectramat®, Ivoclar Vivadent AG) bestrahlt und damit ausgehärtet wurden. Nach der ISO-Norm ISO-4049 (Dentistry - Polymer-based filling, restorative and luting materials) erfolgte die Bestimmung der Biegefestigkeit und des Biege-E-Moduls nach 24 h Lagerung der Prüfkörper bei Raumtemperatur (RT) oder 24 h Lagerung in Wasser bei 37 °C (WL) (Tabelle 2).

**Tabelle 2: Biegefestigkeit und des Biege-E-Moduls der polymerisierten Komposite C1 und C2**

| **Parameter** | **C1** | **C2** |
|---|---|---|
| BF¹⁾, RT | 99,8±8,0 | 66,1±3,4 |
| BF¹⁾, WL | 116,7±7,6 | 72,7±6,6 |
| BM²⁾, RT | 4,60±0,18 | 3,71±0,15 |
| BM²⁾, WL | 5,03±0,20 | 3,92±0,30 |

| | | |
|---|---|---|
| ¹⁾ Biegefestigkeit (BF) in MPa ²⁾ Biege-E-Moduls (BM), GPa | | |

## Patentansprüche

1. Polymerisierbare Zusammensetzung, die
(a) 0,001 bis 5 Gew.-% Acylzinn-Verbindung(en) der allgemeinen Formel (I) als Photoinitiator,
(b) 10 bis 99,999 Gew.-% radikalisch polymerisierbares Bindemittel,
(c) 0 bis 85 Gew.-% Füllstoff und
(d) 0 bis 70 Gew.-% Additiv(e) enthält,
jeweils bezogen auf die Gesamtmasse der Zusammensetzung, wobei die Zusammensetzung als radikalische polymerisierbares Bindemittel mindestens ein mono- oder multifunktionelles (Meth)acrylat oder eine Mischung davon und als Photoinitiator mindestens eine Acylzinn-Verbindung gemäß der allgemeinen Formel (I) enthält, 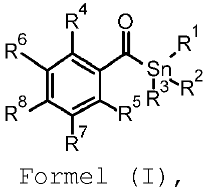 in der
R¹,R²,R³ unabhängig voneinander jeweils eine Gruppe der Formel (II) 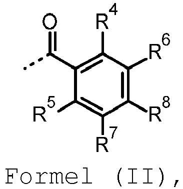 ein aromatischer C₆₋₃₀-Rest, der durch einen oder mehrere cyclische, verzweigte oder vorzugsweise lineare C₁₋₂₀-Alkyl-, C₁₋₂₀-Alkenyl-, C₁₋₂₀-Alkoxy-, C₁₋₂₀-Alkyl-thio- oder C₁₋₂₀-Alkenoxy-Reste substituiert sein kann,
wobei die genannten Substituenten ihrerseits ein- oder mehrfach durch -O-, -S- oder -NR⁹- unterbrochen und/oder durch eine oder mehrere radikalisch polymerisationsfähige Gruppen und/oder Reste R¹⁰ substituiert sein können,
ein cyclischer, verzweigter oder vorzugsweise linearer C₁₋₂₀-Alkyl-, C₁₋₂₀-Alkenyl-, C₁₋₂₀-Alkoxy-, C₁₋₂-Alkyl-thio-, C₁₋₂₀-Alkenoxy- oder C₁₋₂₀-Acyloxy-Rest, der ein- oder mehrfach durch -O-, -S- oder -NR⁹- unterbrochen und/oder durch eine oder mehrere radikalisch polymerisationsfähige Gruppen und/oder Reste R¹⁰ substituiert sein kann, oder ein Benzoyloxy-Rest,
-H, Trimethylsilyl, -OH, Halogen oder -CN sind,
wobei R¹ und R² auch zusammengefasst ein doppelt gebundenes Sauerstoff- oder Schwefelatom darstellen können oder zusammen mit dem Sn-Atom, an das sie gebunden sind, einen aliphatischen gesättigten oder ungesättigten Ring bilden können, der neben dem Sn-Atom 2 bis 6 Kohlenstoffatome und ggf. ein oder mehrere Sauerstoffatome enthält, wobei ein oder mehrere Kohlenstoffatome mit einem doppelt gebundenen Sauerstoffatom substituiert sein können und/oder der Ring mit einem aromatischen Ring anneliert sein kann,
R⁴,R⁵,R⁶,R⁷,R⁸ unabhängig voneinander jeweils -H, ein cyclischer, verzweigter oder vorzugsweise linearer C₁₋₂₀-Alkyl-, C₁₋₂₀-Alkenyl-, C₁₋₂₀-Alkyloxy- oder C₁₋₂₀-Alkenoxy-Rest, der ein- oder mehrfach durch -O-, -S-, oder -NR⁹- unterbrochen und/oder durch eine oder mehrere radikalisch polymerisationsfähige Gruppen und/oder Reste R¹⁰ substituiert sein kann, -OR⁹, Halogen, -SR⁹, -N(R⁹)₂, -CF₃, -CN, -NO₂, -COOR⁹ oder -CONHR⁹ sind,
R⁹ -H oder ein cyclischer, verzweigter oder vorzugsweise linearer C₁₋₂₀-Alkyl- oder C₁₋₂₀-Alkenyl-Rest ist und
R¹⁰ -OH, -CₓF₂ₓ₊₁ mit x = 1 bis 20 oder -[Si(CH₃)₂]_{y}-CH₃ mit y = 1 bis 20 ist.

2. Polymerisierbare Zusammensetzung nach Anspruch 1, bei der jeweils unabhängig voneinander
R¹,R²,R³ unabhängig voneinander jeweils eine Gruppe der Formel (II), ein aromatischer C₆₋₁₅-Rest, der durch einen oder mehrere verzweigte oder vorzugsweise lineare C₁₋₁₂-Alkyl- oder C₁₋₁₂-Alkoxy-Reste substituiert sein kann, wobei die genannten Substituenten ihrerseits ein- oder mehrfach durch -O- unterbrochen und/oder durch eine oder mehrere radikalisch polymerisationsfähige Gruppen und/oder -OH substituiert sein können, ein verzweigter oder vorzugsweise linearer C₁₋₁₂-Alkyl-, C₁₋₁₂-Alkenyl-, C₁₋₁₂-Alkoxy-, C₁₋₁₂-Alkylthio- oder C₁₋₁₂-Acyloxy-Rest, der ein- oder mehrfach durch -O- unterbrochen und/oder durch eine oder mehrere radikalisch polymerisationsfähige Gruppen und/oder -OH substituiert sein kann, ein Benzoyloxy-Rest, Trimethylsilyl, -OH, Halogen oder -CN sind,
wobei R¹ und R² auch zusammengefasst ein doppelt gebundenes Sauerstoff- oder Schwefelatom darstellen können oder zusammen mit dem Sn-Atom, an das sie gebunden sind, einen aliphatischen gesättigten oder ungesättigten Ring bilden können, der neben dem Sn-Atom 2 bis 6, insbesondere 4, Kohlenstoffatome und ggf. ein oder mehrere, insbesondere 2, Sauerstoffatome enthält, wobei ein oder mehrere, insbesondere 2, Kohlenstoffatome mit einem doppelt gebundenen Sauerstoffatom substituiert sein können und/oder der Ring mit einem aromatischen, insbesondere sechsgliedrigen, Ring anneliert sein kann,
R⁴,R⁵,R⁸ unabhängig voneinander jeweils -H, ein verzweigter oder vorzugsweise linearer C₁₋₁₂-Alkyl-, C₁₋₁₂-Alkenyl-, C₁₋₁₂-Alkyloxy- oder C₁₋₁₂-Alkenoxy-Rest sind, der ein- oder mehrfach durch -O- unterbrochen und/oder durch eine oder mehrere radikalisch polymerisationsfähige Gruppen und/oder -OH substituiert sein kann, -OR⁹, Halogen, -SR⁹, -N(R⁹)₂, -CF₃, -CN oder -NO₂ sind,
R⁶,R⁷ unabhängig voneinander jeweils -H oder -F oder ein verzweigter oder vorzugsweise linearer C₁₋₁₂-Alkyl-, C₁₋₁₂-Alkenyl-, C₁₋₁₂-Alkyloxy- oder C₁₋₁₂-Alkenoxy-Rest, der ein- oder mehrfach durch -O-unterbrochen und/oder durch eine oder mehrere radikalisch polymerisationsfähige Gruppen und/oder -OH substituiert sein kann, sind und
R⁹ -H oder Methyl ist.

3. Polymerisierbare Zusammensetzung nach Anspruch 1 oder 2, bei der jeweils unabhängig voneinander
R¹,R²,R³ unabhängig voneinander jeweils eine Gruppe der Formel (II), Phenyl, Trimethylphenyl, ein verzweigter oder vorzugsweise linearer C₁₋₈-Alkyl-, C₁₋₁₂-Alkylthio-oder C₁₋₁₂-Acyloxy-Rest, ein Benzoyloxy-, Vinyl- oder Methacryloyl-Rest, Trimethylsilyl, -OH, -Cl oder -CN sind,
wobei R¹ und R² auch zusammengefasst ein doppelt gebundenes Sauerstoff- oder Schwefelatom darstellen können oder zusammen mit dem Sn-Atom, an das sie gebunden sind, einen Dioxastannepin-Ring bilden können, wobei ein oder vorzugsweise zwei Kohlenstoffatome des Dioxastannepin-Rings mit einem doppelt gebundenen Sauerstoffatom substituiert sein können und/oder der Ring mit einem Benzol-Ring anneliert sein kann,
R⁴,R⁵,R⁸ unabhängig voneinander jeweils -H, ein verzweigter oder vorzugsweise linearer C₁₋₈-Alkyl-Rest sind, der durch 1 bis 3 O-Atome unterbrochen und/oder durch Vinyl substituiert sein kann, -OR⁹, Halogen, -SR⁹, -N(R⁹)₂, -CF₃, -CN oder -NO₂ sind,
R⁶,R⁷ unabhängig voneinander jeweils -H, -F oder ein verzweigter oder vorzugsweise linearer C₁₋₈-Alkyl-Rest sind, der durch 1 bis 3 O-Atome unterbrochen und/oder durch Vinyl substituiert sein kann, und
R⁹ -H oder Methyl ist.

4. Polymerisierbare Zusammensetzung nach einem der Ansprüche 1 bis 3, bei der jeweils unabhängig voneinander
R¹,R²,R³ unabhängig voneinander jeweils eine Gruppe der Formel (II), Phenyl, ein linearer C₁-C₈-Alkylrest oder Trimethylsilyl sind,
R⁴,R⁵,R⁸ unabhängig voneinander jeweils -H, Methyl, -OR⁹ oder -F sind,
R⁶,R⁷ unabhängig voneinander jeweils -H oder -F sind und
R⁹ -H oder Methyl ist.

5. Zusammensetzung nach einem der Ansprüche 1 bis 4, die
(a) 0,001 bis 5 Gew.-% Acylzinn-Verbindung(en) der allgemeinen Formel (I),
(b) 10 bis 50 Gew.-% radikalisch polymerisierbares Bindemittel,
(c) 40 bis 70 Gew.-% Füllstoff und
(d) 0 bis 5 Gew.-% Additiv(e) enthält,
jeweils bezogen auf die Gesamtmasse der Zusammensetzung.

6. Zusammensetzung nach einem der Ansprüche 1 bis 4, die
(a) 0,001 bis 5 Gew.-% Acylzinn-Verbindung(en) der allgemeinen Formel (I),
(b) 10 bis 40 Gew.-% radikalisch polymerisierbares Bindemittel,
(c) 50 bis 85 Gew.-% Füllstoff und
(d) 0 bis 5 Gew.-% Additiv(e) enthält,
jeweils bezogen auf die Gesamtmasse der Zusammensetzung.

7. Zusammensetzung nach einem der Ansprüche 1 bis 4, die
(a) 0,001 bis 5 Gew.-% Acylzinn-Verbindung(en) der allgemeinen Formel (I),
(b) 10 bis 99,989 Gew.-% radikalisch polymerisierbares Bindemittel,
(c) 0 bis 20 Gew.-% nanopartikulären Füllstoff,
(d) 0,01 bis 2 Gew.-% Additiv(e) und
(e) 0 bis 70 Gew.-% Lösungsmittel enthält,
jeweils bezogen auf die Gesamtmasse der Zusammensetzung.

8. Zusammensetzung nach einem der Ansprüche 1 bis 7 zur Verwendung als Dentalwerkstoff.

9. Zusammensetzung nach Anspruch 8 zur intraoralen Anwendung als Zement, Füllungskomposit oder Verblendmaterial.

10. Verwendung einer Zusammensetzung nach einem der Ansprüche 1 bis 7 zur extraoralen Herstellung oder Reparatur von Dentalrestaurationen, Prothesen, künstlichen Zähnen, Inlays, Onlays, Kronen oder Brücken.

11. Verwendung einer Zusammensetzung nach einem der Ansprüche 1 bis 4 zur Herstellung eines Formkörpers durch ein generatives Verfahren.

12. Verwendung einer Zusammensetzung nach einem der Ansprüche 1 bis 4 zur Herstellung von Gehörhilfen, Intraokularlinsen, Kontaktlinsen, Formkörpern, Prototypen, Grünkörpern, Beschichtungen, Lacken, Klebstoffen oder Photoresists.

## Claims

1. Polymerisable composition, which comprises
(a) 0.001 to 5 wt % acyl tin compound(s) of the general Formula (I) as a photoinitiator,
(b) 10 to 99.999 wt % radically polymerisable binder,
(c) 0 to 85 wt % filler and
(d) 0 to 70 wt % additive(s),
in each case relative to the total mass of the composition, wherein the composition comprises, as the radically polymerisable binder, a mono or polyfunctional (meth)acrylate or a mixture thereof and, as the photoinitiator, at least one acyl tin compound according to the general Formula (I), 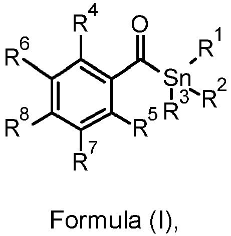 in which
R¹,R²,R³ independently of each other are in each case a group of the Formula (II) 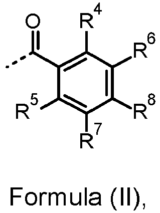 an aromatic C₆₋₃₀ group, which can be substituted by one or more cyclic, branched or preferably linear C₁₋₂₀-alkyl, C₁₋₂₀-alkenyl, C₁₋₂₀-alkoxy, C₁₋₂₀-alkylthio or C₁₋₂₀-alkenoxy groups, wherein the named substituents themselves can be interrupted one or more times by -O-, -S- or -NR⁹- and/or can be substituted by one or more radically polymerisable groups and/or R¹⁰ groups,
a cyclic, branched or preferably linear C₁₋₂₀-alkyl, C₁₋₂₀-alkenyl, C₁₋₂₀-alkoxy, C₁₋₂₀-alkylthio, C₁₋₂₀-alkenoxy or C₁₋₂₀-acyloxy group, which can be interrupted one or more times by -O-, -S- or -NR⁹-and/or can be substituted by one or more radically polymerisable groups and/or R¹⁰ groups, or a benzoyloxy group,
-H, trimethylsilyl, -OH, halogen or -CN,
R⁴,R⁵,R⁶,R⁷,R⁸ wherein R¹ and R², taken together, can also represent a double-bonded oxygen or sulfur atom or, together with the Sn atom to which they are bonded, can form an aliphatic saturated or unsaturated ring that in addition to the Sn atom contains 2 to 6 carbon atoms and optionally one or more oxygen atoms, wherein one or more carbon atoms can be substituted by a double-bonded oxygen atom and/or the ring can be fused with an aromatic ring, independently of each other are in each case -H, a cyclic, branched or preferably linear C₁₋₂₀-alkyl, C₁₋₂₀-alkenyl, C₁₋₂₀-alkyloxy or C₁₋₂₀-alkenoxy group, which can be interrupted one or more times by -O-, -S- or -NR⁹- and/or can be substituted by one or more radically polymerisable groups and/or R¹⁰ groups, or are-OR⁹, halogen, -SR⁹, -N(R⁹)₂, -CF₃, -CN, -NO₂, -COOR⁹ or-CONHR⁹,
R⁹ is -H or a cyclic, branched or preferably linear C₁₋₂₀-alkyl or C₁₋₂₀-alkenyl group and
R¹⁰ is -OH, -CₓF₂ₓ₊₁ with x = 1 to 20 or -[Si(CH₃)₂]_{y}-CH₃ with y = 1 to 20.

2. Polymerisable composition according to claim 1, in which, in each case independently of each other,
R¹,R²,R³ independently of each other are in each case a group of the Formula (II), an aromatic C₆₋₁₅ group, which can be substituted by one or more branched or preferably linear C₁₋₁₂-alkyl or C₁₋₁₂-alkoxy groups, wherein the named substituents themselves can be interrupted one or more times by -O- and/or can be substituted by one or more radically polymerisable groups and/or -OH, or a branched or preferably linear C₁₋₁₂-alkyl, C₁₋₁₂-alkenyl, C₁₋₁₂-alkoxy, C₁₋₁₂-alkylthio or C₁₋₁₂-acyloxy group, which can be interrupted one or more times by -O- and/or can be substituted by one or more radically polymerisable groups and/or -OH, or a benzoyloxy group, trimethylsilyl, -OH, halogen or -CN,
wherein R¹ and R², taken together, can also represent a double-bonded oxygen or sulfur atom or, together with the Sn atom to which they are bonded, can form an aliphatic saturated or unsaturated ring which in addition to the Sn atom contains 2 to 6, in particular 4, carbon atoms and optionally one or more, in particular 2, oxygen atoms, wherein one or more, in particular 2, carbon atoms can be substituted by a double-bonded oxygen atom and/or the ring can be fused with an aromatic, in particular six-membered, ring,
R⁴,R⁵,R⁸ independently of each other are in each case -H, a branched or preferably linear C₁₋₁₂-alkyl, C₁₋₁₂-alkenyl, C₁₋₁₂-alkyloxy or C₁₋₁₂-alkenoxy group, which can be interrupted one or more times by -O-and/or can be substituted by one or more radically polymerisable groups and/or -OH, or -OR⁹, halogen, -SR⁹, -N(R⁹)₂, -CF₃, -CN or-NO₂,
R⁶,R⁷ independently of each other are in each case -H or -F or a branched or preferably linear C₁₋₁₂-alkyl, C₁₋₁₂-alkenyl, C₁₋₁₂-alkyloxy or C₁₋₁₂-alkenoxy group, which can be interrupted one or more times by -O- and/or can be substituted by one or more radically polymerisable groups and/or -OH, and
R⁹ is -H or methyl.

3. Polymerisable composition according to claim 1 or 2, in which, in each case independently of each other,
R¹,R²,R³ independently of each other are in each case a group of the Formula (II), phenyl, trimethylphenyl, a branched or preferably linear C₁₋₈-alkyl, C₁₋₁₂-alkylthio or C₁₋₁₂-acyloxy group, a benzoyloxy, vinyl or methacryloyl group, trimethylsilyl, -OH, -CI or -CN,
wherein R¹ and R², taken together, can also represent a double-bonded oxygen or sulfur atom or, together with the Sn atom to which they are bonded, can form a dioxastannepin ring, wherein one or preferably two carbon atoms of the dioxastannepin ring can be substituted by a double-bonded oxygen atom and/or the ring can be fused with a benzene ring,
R⁴,R⁵,R⁸ independently of each other are in each case -H, a branched or preferably linear C₁₋₈-alkyl group, which can be interrupted by 1 to 3 O atoms and/or can be substituted by vinyl, or -OR⁹, halogen, -SR⁹, -N(R⁹)₂, -CF₃, -CN or -NO₂,
R⁶,R⁷ independently of each other are in each case -H, -F or a branched or preferably linear C₁₋₈-alkyl group, which can be interrupted by 1 to 3 O atoms and/or can be substituted by vinyl, and
R⁹ is -H or methyl.

4. Polymerisable composition according to one of claims 1 to 3, in which, in each case independently of each other,
R¹,R²,R³ independently of each other are in each case a group of the Formula (II), phenyl, a linear C₁-C₈-alkyl group or trimethylsilyl,
R⁴,R⁵,R⁸ independently of each other are in each case -H, methyl, -OR⁹ or-F,
R⁶,R⁷ independently of each other are in each case -H or -F and
R⁹ is -H or methyl.

5. Composition according to one of claims 1 to 4, which comprises
(a) 0.001 to 5 wt % acyl tin compound(s) of the general Formula (I),
(b) 10 to 50 wt % radically polymerisable binder,
(c) 40 to 70 wt % filler and
(d) 0 to 5 wt % additive(s),
in each case relative to the total mass of the composition.

6. Composition according to one of claims 1 to 4, which comprises
(a) 0.001 to 5 wt % acyl tin compound(s) of the general Formula (I),
(b) 10 to 40 wt % radically polymerisable binder,
(c) 50 to 85 wt % filler and
(d) 0 to 5 wt % additive(s),
in each case relative to the total mass of the composition.

7. Composition according to one of claims 1 to 4, which comprises
(a) 0.001 to 5 wt % acyl tin compound(s) of the general Formula (I),
(b) 10 to 99.989 wt % radically polymerisable binder,
(c) 0 to 20 wt % nanoparticulate filler,
(d) 0.01 to 2 wt % additive(s) and
(e) 0 to 70 wt % solvent,
in each case relative to the total mass of the composition.

8. Composition according to one of claims 1 to 7 for use as a dental material.

9. Composition according to claim 8 for intraoral use as cement, filling composite or veneering material.

10. Use of a composition according to one of claims 1 to 7 for the extraoral production or repair of dental restorations, prostheses, artificial teeth, inlays, onlays, crowns or bridges.

11. Use of a composition according to one of claims 1 to 4 for the production of a shaped body by a generative process.

12. Use of a composition according to one of claims 1 to 4 for the production of hearing aids, intraocular lenses, contact lenses, shaped bodies, prototypes, green bodies, coatings, varnishes, adhesives or photoresists.

## Revendications

1. Composition polymérisable, qui contient
a) de 0,001 à 5 % en poids d'un ou de composé(s) de type acyl-étain, de formule générale (I), en tant que photoamorceur,
b) de 10 à 99,999 % en poids de liant polymérisable par voie radicalaire,
c) de 0 à 85 % en poids de charge,
d) et de 0 à 70 % en poids d'adjuvant(s),
par rapport, pour chaque composant, au poids total de la composition, laquelle composition contient, en tant que liant polymérisable par voie radicalaire, au moins un acrylate ou méthacrylate monofonctionnel ou polyfonctionnel ou un mélange de tels composés, et en tant que photoamorceur, au moins un composé de type acyl-étain, de formule générale (I) : 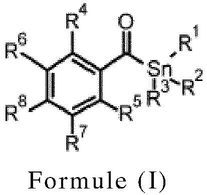 dans laquelle
- les symboles R¹, R² et R³ représentent chacun, indépendamment l'un des autres,
- un groupe de formule (II) 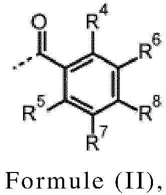
- un groupe aromatique en C₆₋₃₀, qui peut porter, en tant que substituants(s), un ou plusieurs groupe(s) alkyle en C₁₋₂₀, alcényle en C₁₋₂₀, alcoxy en C₁₋₂₀, alkylthio en C₁₋₂₀ ou alcényloxy en C₁₋₂₀, cyclique(s), ramifié(s) ou de préférence linéaire(s), lesquels substituants peuvent de leur côté présenter une chaîne interrompue une ou plusieurs fois par un chaînon symbolisé par -O-, -S- ou -NR⁹-, et/ou porter en tant que substituant(s) un ou plusieurs groupe(s) polymérisable(s) par voie radicalaire et/ou un ou plusieurs groupe(s) symbolisé(s) par R¹⁰,
- un groupe alkyle en C₁₋₂₀, alcényle en C₁₋₂₀, alcoxy en C₁₋₂₀, alkylthio en C₁₋₂₀, alcényloxy en C₁₋₂₀ ou acyloxy en C₁₋₂₀, cyclique, ramifié ou de préférence linéaire, dont la chaîne peut être interrompue une ou plusieurs fois par un chaînon symbolisé par -O-,
- S- ou -NR⁹-, et/ou qui peut porter en tant que substituant(s) un ou plusieurs groupe(s) polymérisable(s) par voie radicalaire et/ou un ou plusieurs groupe(s) symbolisé(s) par R¹⁰,
- ou un groupe benzoyloxy, un atome d'hydrogène, un groupe triméthyl-silyle, un groupe hydroxyle, un atome d'halogène ou un groupe cyano,
étant entendu que les symboles R¹ et R² peuvent aussi représenter conjointement un atome d'oxygène ou de soufre lié par double liaison, ou représenter des entités formant, conjointement avec l'atome d'étain auquel elles sont liées, un cycle aliphatique saturé ou insaturé comportant, outre l'atome d'étain, de 2 à 6 atomes de carbone et un ou plusieurs éventuel(s) atome(s) d'oxygène, dans lequel cycle un ou plusieurs atome(s) de carbone peuvent porter, en tant que substituant, un atome d'oxygène lié par double liaison, et/ou lequel cycle peut être condensé avec un cycle aromatique,
- et les symboles R⁴, R⁵, R⁶, R⁷ et R⁸ représentent chacun, indépendamment les uns des autres,
- un atome d'hydrogène,
- un groupe alkyle en C₁₋₂₀, alcényle en C₁₋₂₀, alcoxy en C₁₋₂₀ ou alcényloxy en C₁₋₂₀, cyclique, ramifié ou de préférence linéaire, dont la chaîne peut être interrompue une ou plusieurs fois par un chaînon symbolisé par -O-, -S- ou -NR⁹-, et/ou qui peut porter en tant que substituant(s) un ou plusieurs groupe(s) polymérisable(s) par voie radicalaire et/ou un ou plusieurs groupe(s) symbolisé(s) par R¹⁰,
- ou un atome d'halogène ou un groupe symbolisé par -OR⁹, -SR⁹, -N(R⁹)₂, -CF₃, -CN, -NO₂, -COOR⁹ ou -CONHR⁹,
étant entendu que
- R⁹ représente un atome d'hydrogène ou un groupe alkyle en C₁₋₂₀ ou alcényle en C₁₋₂₀, cyclique, ramifié ou de préférence linéaire,
- et R¹⁰ représente un groupe hydroxyle ou un groupe symbolisé par -CₓF₂ₓ₊₁ où x vaut de 1 à 20 ou par -[Si(CH₃)₂]_{y}-CH₃ où y vaut de 1 à 20.

2. Composition polymérisable conforme à la revendication 1, dans laquelle, indépendamment :
- les symboles R¹, R² et R³ représentent chacun, indépendamment l'un des autres,
- un groupe de formule (II),
- un groupe aromatique en C₆₋₁₅, qui peut porter, en tant que substituants(s), un ou plusieurs groupe(s) alkyle en C₁₋₁₂ ou alcoxy en C₁₋₁₂, ramifié(s) ou de préférence linéaire(s), lesquels substituants peuvent de leur côté présenter une chaîne interrompue une ou plusieurs fois par un chaînon symbolisé par -O-, et/ou porter en tant que substituant(s) un ou plusieurs groupe(s) polymérisable(s) par voie radicalaire et/ou un ou plusieurs groupe(s) hydroxyle,
- un groupe alkyle en C₁₋₁₂, alcényle en C₁₋₁₂, alcoxy en C₁₋₁₂, alkylthio en C₁₋₁₂ ou acyloxy en C₁₋₁₂, ramifié ou de préférence linéaire, dont la chaîne peut être interrompue une ou plusieurs fois par un chaînon symbolisé par -O-, et/ou qui peut porter en tant que substituant(s) un ou plusieurs groupe(s) polymérisable(s) par voie radicalaire et/ou un ou plusieurs groupe(s) hydroxyle,
- ou un groupe benzoyloxy, triméthyl-silyle, hydroxyle ou cyano, ou un atome d'halogène,
étant entendu que les symboles R¹ et R² peuvent aussi représenter conjointement un atome d'oxygène ou de soufre lié par double liaison, ou représenter des entités formant, conjointement avec l'atome d'étain auquel elles sont liées, un cycle aliphatique saturé ou insaturé comportant, outre l'atome d'étain, de 2 à 6, en particulier 4, atomes de carbone et un ou plusieurs, en particulier 2, éventuel(s) atome(s) d'oxygène, dans lequel cycle un ou plusieurs, en particulier 2, atome(s) de carbone peuvent porter, en tant que substituant, un atome d'oxygène lié par double liaison, et/ou lequel cycle peut être condensé avec un cycle aromatique, en particulier à 6 chaînons,
- les symboles R⁴, R⁵ et R⁸ représentent chacun, indépendamment l'un des autres,
- un atome d'hydrogène,
- un groupe alkyle en C₁₋₁₂, alcényle en C₁₋₁₂, alcoxy en C₁₋₁₂ ou alcényloxy en C₁₋₁₂, ramifié ou de préférence linéaire, dont la chaîne peut être interrompue une ou plusieurs fois par un chaînon symbolisé par -O-, et/ou qui peut porter en tant que substituant(s) un ou plusieurs groupe(s) polymérisable(s) par voie radicalaire et/ou un ou plusieurs groupe(s) hydroxyle,
- ou un atome d'halogène ou un groupe symbolisé par -OR⁹, -SR⁹, -N(R⁹)₂, -CF₃, -CN ou -NO₂,
- et les symboles R⁶ et R⁷ représentent chacun, indépendamment l'un de l'autre,
- un atome d'hydrogène ou de fluor,
- ou un groupe alkyle en C₁₋₁₂, alcényle en C₁₋₁₂, alcoxy en C₁₋₁₂ ou alcényloxy en C₁₋₁₂, ramifié ou de préférence linéaire, dont la chaîne peut être interrompue une ou plusieurs fois par un chaînon symbolisé par -O-, et/ou qui peut porter en tant que substituant(s) un ou plusieurs groupe(s) polymérisable(s) par voie radicalaire et/ou un ou plusieurs groupe(s) hydroxyle,
étant entendu que
- R⁹ représente un atome d'hydrogène ou un groupe méthyle.

3. Composition polymérisable conforme à la revendication 1 ou 2, dans laquelle, indépendamment :
- les symboles R¹, R² et R³ représentent chacun, indépendamment l'un des autres, un groupe de formule (II), un groupe phényle ou triméthyl-phényle, un groupe alkyle en C₁₋₈, alkylthio en C₁₋₁₂ ou acyloxy en C₁₋₁₂, ramifié ou de préférence linéaire, ou un groupe benzoyloxy, vinyle, méthacryloyle, triméthyl-silyle, hydroxyle ou cyano, ou un atome de chlore,
étant entendu que les symboles R¹ et R² peuvent aussi représenter conjointement un atome d'oxygène ou de soufre lié par double liaison, ou représenter des entités formant, conjointement avec l'atome d'étain auquel elles sont liées, un cycle dioxastannépine, dans lequel cycle dioxastannépine un ou de préférence deux atome(s) de carbone peuvent porter, en tant que substituant, un atome d'oxygène lié par double liaison, et/ou lequel cycle peut être condensé avec un cycle benzénique,
- les symboles R⁴, R⁵ et R⁸ représentent chacun, indépendamment l'un des autres, un atome d'hydrogène, un groupe alkyle en C₁₋₈, ramifié ou de préférence linéaire, dont la chaîne peut être interrompue par 1 à 3 atomes d'oxygène et/ou qui peut porter en tant que substituant un groupe vinyle, ou un atome d'halogène ou un groupe symbolisé par -OR⁹, -SR⁹, -N(R⁹)₂, -CF₃, -CN ou -NO₂,
- et les symboles R⁶ et R⁷ représentent chacun, indépendamment l'un de l'autre, un atome d'hydrogène ou de fluor ou un groupe alkyle en C₁₋₈, ramifié ou de préférence linéaire, dont la chaîne peut être interrompue par 1 à 3 atomes d'oxygène et/ou qui peut porter en tant que substituant un groupe vinyle,
étant entendu que R⁹ représente un atome d'hydrogène ou un groupe méthyle.

4. Composition conforme à l'une des revendications 1 à 3, dans laquelle, indépendamment,
- les symboles R¹, R² et R³ représentent chacun, indépendamment l'un des autres, un groupe de formule (II), un groupe phényle, un groupe alkyle linéaire en C₁-C₈, ou un groupe triméthyl-silyle,
- les symboles R⁴, R⁵ et R⁸ représentent chacun, indépendamment l'un des autres, un atome d'hydrogène, un groupe méthyle, un groupe symbolisé par -OR⁹, ou un atome de fluor,
- et les symboles R⁶ et R⁷ représentent chacun, indépendamment l'un de l'autre, un atome d'hydrogène ou de fluor,
étant entendu que R⁹ représente un atome d'hydrogène ou un groupe méthyle.

5. Composition conforme à l'une des revendications 1 à 4, qui contient :
a) de 0,001 à 5 % en poids d'un ou de composé(s) de type acyl-étain, de formule générale (I),
b) de 10 à 50 % en poids de liant polymérisable par voie radicalaire,
c) de 40 à 70 % en poids de charge,
d) et de 0 à 5 % en poids d'adjuvant(s),
par rapport, pour chaque composant, au poids total de la composition.

6. Composition conforme à l'une des revendications 1 à 4, qui contient :
a) de 0,001 à 5 % en poids d'un ou de composé(s) de type acyl-étain, de formule générale (I),
b) de 10 à 40 % en poids de liant polymérisable par voie radicalaire,
c) de 50 à 85 % en poids de charge,
d) et de 0 à 5 % en poids d'adjuvant(s),
par rapport, pour chaque composant, au poids total de la composition.

7. Composition conforme à l'une des revendications 1 à 4, qui contient
a) de 0,001 à 5 % en poids d'un ou de composé(s) de type acyl-étain, de formule générale (I),
b) de 10 à 99,989 % en poids de liant polymérisable par voie radicalaire,
c) de 0 à 20 % en poids de charge en particules nanométriques,
d) de 0,01 à 2 % en poids d'adjuvant(s),
d) et de 0 à 70 % en poids de solvant,
par rapport, pour chaque composant, au poids total de la composition.

8. Composition conforme à l'une des revendications 1 à 7, pour utilisation en tant que matériau dentaire.

9. Composition conforme à la revendication 8, pour application intrabuccale en tant que ciment, matériau composite d'obturation ou matériau de placage.

10. Utilisation d'une composition conforme à l'une des revendications 1 à 7 pour la fabrication ou réparation extrabuccale de restaurations dentaires, de prothèses, de dents artificielles, d'incrustations « inlay » ou « onlay », de couronnes ou de bridges.

11. Utilisation d'une composition conforme à l'une des revendications 1 à 4 pour la fabrication d'un corps façonné selon un procédé génératif.

12. Utilisation d'une composition conforme à l'une des revendications 1 à 4 pour la fabrication d'aides auditives, de lentilles intraoculaires, de lentilles de contact, de corps façonnés, de prototypes, de corps non-cuits, d'enduits, de vernis, d'adhésifs ou de résines photosensibles.
